(19) Europäisches Patentamt
European Patent Office
Office européen des brevets

(11) Veröffentlichungsnummer: **0 193 055**
**B1**

(12) **EUROPÄISCHE PATENTSCHRIFT**

(45) Veröffentlichungstag der Patentschrift: **16.05.90**

(51) Int. Cl.[5]: **C 07 F 15/00,** A 61 K 31/28

(21) Anmeldenummer: **86101940.4**

(22) Anmeldetag: **15.02.86**

(54) **Tumorhemmende (1,2-Diphenyl-ethylendiamin)-platin(II)-Komplexe.**

(30) Priorität: **23.02.85 DE 3506507**

(43) Veröffentlichungstag der Anmeldung:
**03.09.86 Patentblatt 86/36**

(45) Bekanntmachung des Hinweises auf die Patenterteilung:
**16.05.90 Patentblatt 90/20**

(84) Benannte Vertragsstaaten:
**AT BE CH DE FR GB IT LI LU NL SE**

(56) Entgegenhaltungen:
**DE-A-3 405 611**

(73) Patentinhaber: **ASTA Pharma AG**
**Weismüllerstrasse 45**
**D-6000 Frankfurt am Main 1 (DE)**

(72) Erfinder: **Schönenberger, Helmut, Prof.Dr.**
**Ahornstrasse 14**
**D-8401 Pentling (DE)**
Erfinder: **Von Angerer, Erwin, Dr.Dipl.-Chem.**
**Regensburger Strasse 6**
**D-8401 Grasslfing (DE)**
Erfinder: **Karl, Johann, Dipl.-Chem.**
**Krankenhausstrasse 67**
**D-8406 Sünching (DE)**
Erfinder: **Jennerwein, Margaretha, Dipl.-Chem.**
**Brückmaierweg 22**
**D-8400 Regensburg (DE)**
Erfinder: **Engel, Jürgen, Dr.**
**Erlenweg 3**
**D-8755 Alzenau (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 193 055 B1

**Beschreibung**

Aus der deutschen Offenlegungsschrift 34 05 611 sind antitumorwirksame Verbindungen bekannt. Es handelt sich hierbei um (1,2-Diphenyl-ethylendiamin)-platin(II)-Komplexverbindungen der allgemeinen Formel

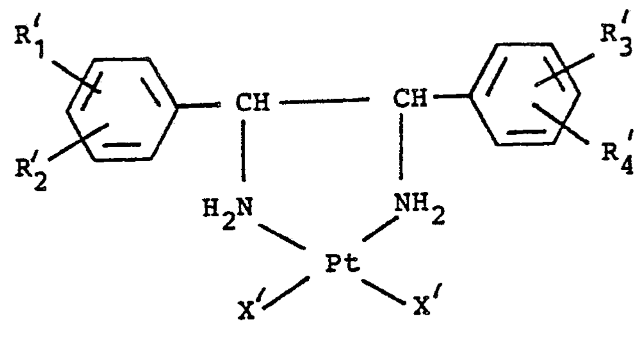

worin die Reste $R'_1$, $R'_2$, $R'_3$ und $R'_4$ gleich oder verschieden sind und Wasserstoff, Hydroxygruppen, $C_1$—$C_6$-Alkoxygruppen, gegebenenfalls durch Halogenatome oder $C_1$—$C_4$-Alkansulfonyloxygruppen substituierte $C_2$—$C_6$-Alkanoyloxygruppen oder $C_3$—$C_6$-Alkenoyloxygruppen bedeuten, wobei mindestens einer der Reste $R'_1$, $R'_2$, $R'_3$ oder $R'_4$ kein Wasserstoffatom ist, und X' für das Äquivalent eines physiologisch verträglichen Anions steht.

Die Erfindung betrifft neue (1,2-Diphenyl-ethylendiamin)-platin(II)-Komplexe, die als Arzneimittelwirkstoffe verwendbar sind.

Die erfindungsgemäßen Verbindungen werden durch die Formel

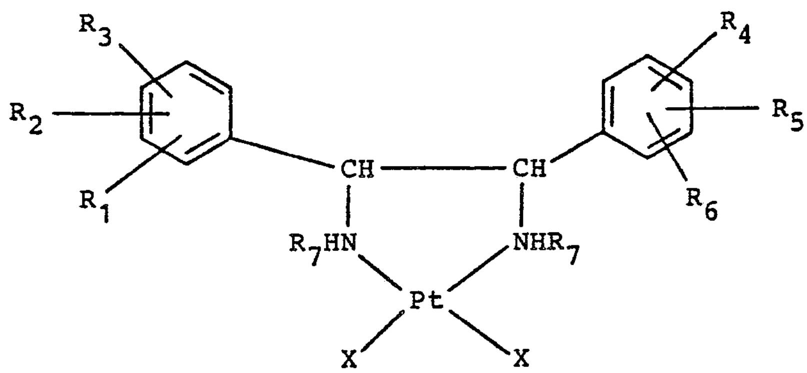

I

gekennzeichnet, worin $R_7$ Wasserstoff oder $C_1$—$C_6$-Alkyl bedeutet und $R_2$ ein Halogenatom ist und die Reste $R_1$, $R_3$, $R_4$, $R_5$ und $R_6$ gleich oder verschieden sind und Wasserstoff, Halogen, Trihalogenmethyl, $C_1$—$C_6$-Alkyl, Hydroxy, $C_1$—$C_6$-Alkoxy oder eine gegebenenfalls durch Halogen oder $C_1$—$C_4$-Alkansulfonyloxy substituierte $C_2$—$C_6$-Alkanoyloxygruppe bedeuten, und X für das Äquivalent eines physiologisch verträglichen Anions steht.

Die neuen erfindungsgemäßen Verbindungen besitzen eine ausgeprägte Antitumorwirkung bei guter Verträglichkeit. Die Wirkung zeigt sich insbesondere bei folgenden Tier- und Zellkultuemodellen: Mäuseleukämie P 388, hormonunabhängige, menschliche Brustkrebszellinie MDA—MB 231, hormonabhängige menschliche Brustkrebszellinie MCF 7, hormonabhängiges Mäusemammacarcinom MXT, DMBA-induziertes hormonabhängiges Rattenmammacarcinom (DMBA = Dimethylbenz[a]-anthracen).

Die erfindungsgemäßen Verbindungen verhindern beziehungsweise hemmen sowohl das Wachstum von vorhandenen Tumorzellen als auch die Bildung neuer Tumorzellen; weiterhin zerstören sie vorhandene Tumorzellen beziehungsweise führen zu deren Rückbildung und verhindern beziehungsweise schwächen die Bildung von Metastasen. Besonders wichtige erfindungsgemäße Verbindungen werden durch die folgende Formel beschrieben:

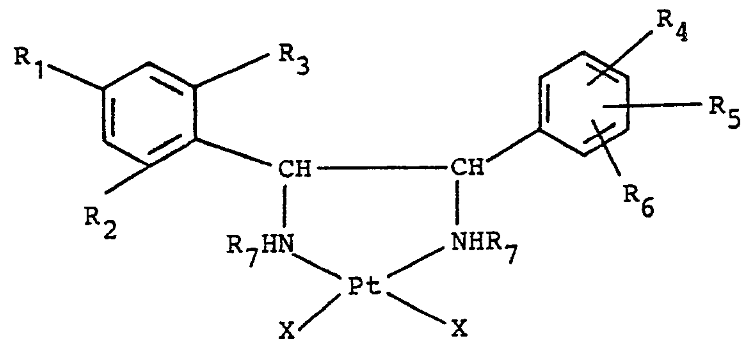

IA

In dieser Formel kommen als Bedeutungen für die einzelnen Reste $R_1$ bis $R_7$ beispielsweise die folgenden in Frage:

$R_1$ = OH, $C_1$—$C_6$-Alkoxy, eine gegebenenfalls durch Halogen oder $C_1$—$C_4$-Alkansulfonyloxy substituierte $C_2$—$C_6$-Alkanoyloxygruppe;

$R_2$ = Halogen;

$R_3$ = Wasserstoff oder Halogen;

$R_4$, $R_5$, $R_6$ = H, Halogen, Trihalogenmethyl, $C_1$—$C_6$-Alkyl, OH, $C_1$—$C_6$-Alkoxy, eine gegebenenfalls durch Halogen oder $C_1$—$C_4$-Alkansulfonyloxy substituierte $C_2$—$C_6$-Alkanoyloxygruppe; zum Beispiel ist $R_4$ ein Halogenatom (F, Cl, Br), vorzugsweise in 4-Stellung, und $R_5$ und $R_6$ sind Wasserstoff oder $R_4$ hat dieselbe Bedeutung wie $R_2$ und befindet sich in 4-Stellung und $R_1$, $R_3$, $R_5$ und $R_6$ bedeuten Halogen (vorzugsweise jeweils in den 2,6-Stellungen).

$R_7$ = H oder $C_1$—$C_6$-Alkyl.

Bei diesen Verbindungen ist zum Beispiel die Mesoform besonders stark wirksam. Weitere wichtige erfindungsgemäße Verbindungen werden durch die folgende Formel IB beschrieben:

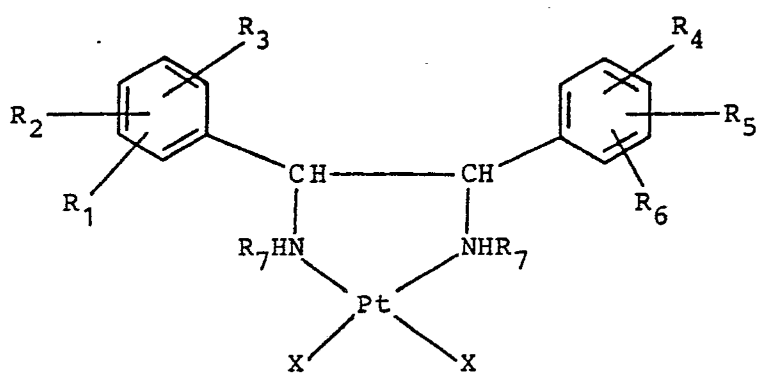

IB

In dieser Formel bedeutet $R_2$ ein Halogenatom (zum Beispiel F, Cl oder Br vorzugsweise in 3- oder 4-Stellung), die anderen Reste $R_1$ und $R_3$, die gleich oder verschieden sein können, bedeuten Wasserstoff oder ein Halogenatom, während die Reste $R_4$, $R_5$ und $R_6$ gleich oder verschieden sind und entweder sämtlich Wasserstoff bedeuten oder ebenfalls Wasserstoff und Halogen (zum Beispiel F, Cl oder Brom) darstellen, wobei die Halogenatome sich vorzugsweise in 3- oder 4-Stellung befinden und vorzugsweise mindestens einer der Reste $R_4$, $R_5$ oder $R_6$ ein Halogenatom darstellt. Insbesondere kommen auch hier solche Verbindungen in Frage, wo die beiden Phenylringe durch die gleichen Substituenten symmetrisch substituiert sind.

Bei diesen Verbindungen sind im allgemeinen die Racemate am stärksten wirksam.

Die folgenden Angaben betreffen bevorzugte Ausgestaltungen der Erfindung:

Die $C_1$—$C_6$-Alkylgruppen, die vorkommenden Alkoxygruppen und die $C_2$—$C_6$-Alkanoyloxygruppen können gerade oder verzweigt sein und bestehen vorzugsweise aus 1 bis 4 C-Atomen. Als Alkanoyloxygruppe kommt insbesondere die Acetoxygruppe in Frage. Die Alkanoyloxygruppen können einen oder mehrere (zum Beispiel 1 bis 6, insbesondere 1 bis 3) gleiche oder verschiedene Halogenatome enthalten. Insbesondere befinden sich 1, 2 oder 3 Halogenatome an einem C-Atom vorzugsweise an dem α-C-Atom. Ferner können sich die Halogenatome sowie der Alkansulfonyloxyrest vorzugsweise in β-Stellung des Alkanoyloxyrestes befinden. Beispielsweise handelt es sich um den Methan- oder Ethansulfonyloxyrest. Als Halogensubstituenten kommen insbesondere Fluor, Chlor und/oder Brom in Frage. Bei der Trihalogenmethylgruppe handelt es sich insbesondere um Trifluormethyl. Die Substituenten $R_4$, $R_5$ und $R_6$ befinden sich vorzugsweise in 4- und/oder 2-Stellung des Phenylrestes. Falls einer der Reste $R_4$, $R_5$ oder $R_6$ Wasserstoff ist, können sich die 2 anderen Substituenten vorzugsweise auch in der 2,4-Stellung des Phenylrestes befinden. Falls einer oder mehrere der Reste $R_1$, $R_3$, $R_4$, $R_5$, $R_6$ oder $R_7$ eine

Alkylgruppe bedeuten, handelt es sich vorzugsweise um eine Methylgruppe, eine Ethylgruppe, eine Isopropylgruppe oder eine Butylgruppe. Die Bedeutung von $R_7$ an den beiden Stickstoffatomen des platinhaltigen Fünfringes kann dieselbe sein; es ist aber auch möglich, daß $R_7$ an dem einen N-Atom Wasserstoff ist und $R_7$ an dem anderen N-Atom eine $C_1$—$C_6$-Alkylgruppe darstellt.

Die Reste X stellen die bekannten und üblichen physiologisch verträglichen und pharmazeutisch verwendbaren Anionen ein- oder mehrwertiger Säuren dar. Insbesondere kommen beispielsweise die Anionen folgender Säuren in Frage: HBr, HCl, HJ, HF, $HNO_3$, $H_2SO_4$ ($SO_4^{--}$); $H_3PO_4$ ($HPO_4^{--}$); $H_2CO_3$, ($CO_3^{--}$); Kampfersulfonsäure, aliphatische oder aromatische Sulfonsäuren, beispielsweise $C_1$—$C_6$-Alkylsulfonsäuren (zum Beispiel Methansulfonsäure, Ethan-, Propan- oder Hexansulfonsäure), Benzol- oder Naphthalinsulfonsäure, die gegebenenfalls ein- oder zweifach durch Methylgruppen substituiert sind (Toluolsulfonsäure, insbesondere o- oder p-Toluolsulfonsäure); aliphatische $C_1$—$C_4$-Monocarbonsäuren, die gegebenenfalls ein-, zwei- oder dreifach durch Halogenatome (insbesondere Cl, F) substituiert sind (zum Beispiel Ameisensäure, Essigsäure, Propionsäure, Chloressigsäure, Dichloressigsäure, Trifluoressigsäure, Trichloressigsäure); aliphatische $C_2$—$C_{11}$-Dicarbonsäuren, die gegebenenfalls eine Doppelbindung enthalten (zum Beispiel Oxalsäure, Malonsäure, 2-Amino-Malonsäure, Malonsäure, welche in 2-Stellung durch eine Benzylgruppe oder eine oder zwei $C_1$—$C_4$-Alkylgruppen substituiert ist, Maleinsäure, Fumarsäure, Bernsteinsäure); aliphatische Monohydroxy- und Dihydroxy-Monocarbonsäuren mit 2 bis 6, insbesondere 2 bis 3 Kohlenstoffatomen, wobei es sich vorzugsweise um α-Monohydroxycarbonsäuren handelt wie Milchsäure, Glycerinsäure oder Glykolsäure; aliphatische Monohydroxy- und Dihydroxy-, Di- und Tricarbonsäuren mit 3 bis 8 Kohlenstoffatomen, insbesondere 3 bis 6 Kohlenstoffatomen wie Äpfelsäure, Weinsäure, Malonsäure, die an dem mittelständigen C-Atomen durch eine Hydroxygruppe und gegebenenfalls eine $C_1$—$C_4$-Alkylgruppe substituiert sein kann, Isozitronensäure oder Zitronensäure; Phthalsäure, die gegebenenfalls durch eine Carboxygruppe (insbesondere in 4-Stellung) substituiert ist; Gluconsäure; Glucuronsäure; die natürlichen α-Aminosäuren (zum Beispiel L-Asparaginsäure); 1,1-Cyclobutandicarbonsäure; Organophosphorsäuren, wie Aldose- und Ketosephosphorsäuren (beispielsweise die entsprechenden Mono- und Diphosphorsäuren) zum Beispiel Aldose-6-phosphorsäuren wie D- oder L-Glucose-6-phosphor-säure, α-D-Glucose-1-phosphorsäure, D-Fructose-6-phosphorsäure, D-Galactose-6-phosphorsäure, D-Ribose-5-phosphorsäure, D-Fructose-1,6-diphosphorsäuren; Glycerionphosphorsäuren (wobei der Phosphorsäurerest an einem der endständigen oder an dem mittelständigen Glycerinsauerstoffatom gebunden ist) wie α-D,L-Glycerin-phosphorsäure, β-Glycerinphosphorsäure; N-Phosphonoacetyl-Asparaginsäure.

Als Säuren für die Anionen X kommen weiterhin in Frage: Aromatische Carbonsäuren, die eine oder mehrere Carboxygruppen enthalten sowie außerdem noch $C_1$—$C_4$-Alkoxygruppen und/oder Hydroxygruppen. Falls sich mehrere Carboxygruppen an dem aromatischen Rest (zum Beispiel Benzolring) befinden, stehen mindestens 2 Carboxygruppen bevorzugt zueinander in Nachbarstellung. Falls der Benzolring zum Beispiel 4 oder 5 Carboxygruppen enthält, können Komplexe entstehen, die pro 1 Mol des Benzolcarbonsäureanions 2 Mol der Platin-Komponente enthalten. 2 benachbarte Carboxygruppen neutralisieren jeweils 1 Mol der Platinkomponente, so daß zum Beispiel im Falle der Benzolpentacarbonsäure die 1- und 2-ständigen sowie die 4- und 5-ständigen Carboxygruppen jeweils 1 Mol der Platinkomponente absättigen (zusammen also 2 Mol), während die freie 3-ständige Carboxygruppe frei oder in der Salzform mit einem physiologisch verträglichen Kation (zum Beispiel Alkalikation, insbesondere Natriumkation) vorliegt. Dies gilt ganz allgemein, wenn die Anionen X noch zusätzliche Säurefunktionen besitzen, die nicht für die Absättigung des Platins gebraucht werden. Das Analoge gilt für den Fall der Benzolhexacarbonsäure, wobei hier gegebenenfalls 1 Mol dieser Säure 3 Mol der Platinkomponente absättigen kann.

Beispiele für solche Säuren sind: Benzolmonocarbonsäure, Benzoldicarbonsäuren, Benzoltricarbonsäuren (zum Beispiel Trimellithsäure), Benzoltetracarbonsäuren, Benzolpentacarbonsäure, Benzolhexacarbonsäure; Syringasäure, Orotsäure.

Ebenfalls kommen als Säuren, die die Anionen X bilden, Aminosäuren beziehungsweise Aminosäurederivate, deren basische Aminogruppe durch eine Säuregruppe neutralisiert ist, in Frage. Es handelt sich hierbei zum Beispiel um Aminosäuren der folgenden Struktur:

$$\begin{array}{c} R'—CH—CO_2H \\ | \\ NH_2 \end{array}$$

worin R' Wasserstoff, einen Phenylrest, einen Indolyl-(3)-methylrest, Imidazolyl-(4)-methylrest, eine $C_1$—$C_{10}$-Alkylgruppe oder eine $C_1$—$C_{10}$-Alkylgruppe, die durch eine Hydroxygruppe, eine Carboxygruppe, eine $C_1$—$C_6$-Alkoxygruppe, eine Mercaptogruppe, eine $C_1$—$C_6$-Alkylthiogruppe, eine Phenylgruppe, eine Hydroxyphenylgruppe, eine $C_2$—$C_6$-Alkanoylaminogruppe oder eine $C_1$—$C_6$-Alkoxycarbonylgruppe substituiert ist, bedeutet.

Die basische Aminogruppe in 2-Stellung ist hierbei durch eine übliche Aminosäureschutzgruppe neutralisiert (acyliert), beispielsweise durch einen $C_2$—$C_6$-Alkanoyl rest oder den Butyloxycarbonylrest. Falls in der obigen Formel R' eine Alkylgruppe ist, handelt es sich vorzugsweise um eine $C_1$—$C_6$-Alkylgruppe, die zum Beispiel in 2-, 3-, 4-, 5- oder 6-Stellung (Zählung beginnt an der Verknüpfungsstelle

des Alkylrestes mit dem Restmolekül) eine $C_2$—$C_6$-Alkanoylaminogruppe, einen Imidazolyl-(4)-methylrest oder einen Indolyl-(3)-methylrest enthält. Einzelbeispiele für solche Aminosäuren sind: Leucin (vorzugsweise D- und L-Form), Valin (vorzugsweise D- und L-Form), Phenylalanin (vorzugsweise D- und L-Form), Phenylglycin (vorzugsweise D- und L-Form), Alanin (vorzugsweise D- und L-Form), Isoleucin (vorzugsweise D- und L-Form), Asparagin (vorzugsweise D- und L-Form, Lysin (vorzugsweise D- und L-Form), Tryptophan (vorzugsweise D- und L-Form), Tyrosin (vorzugsweise D- und L-Form), Ortnithin (vorzugsweise D- und L-Form).

Hierbei sind die basischen Aminogruppen durch eine übliche Acylaminoschutzgruppe blockiert, insbesondere durch die Acetylgruppe oder die Butyloxycarbonylgruppe.

Die Formel I umfaßt auch die möglichen Enantiomere und Diastereomere. Falls die Verbindungen Racemate sind, können diese in an sich bekannter Weise, zum Beispiel mittels einer optisch aktiven Säure in die optisch aktiven Isomere gespalten werden. Es ist aber auch möglich, von vornherein enantiomere oder gegebenenfalls auch diastereomere Ausgangsstoffe einzusetzen, wobei dann als Endprodukt eine entsprechende reine optisch aktive beziehungsweise diastereomere Verbindung erhalten wird. Unabhängig von der Struktur der Reste X besitzt bereits der 1,2-Diphenyl-ethylendiamin-Teil 2 asymmetrische Kohlenstoffatome und kann daher in der Racemat-Form oder in links- beziehungsweise rechtsdrehender Form oder in der meso-Form vorliegen. Zusätzliche Formen können durch verschiedene enantiomere beziehungsweise diastereomere Formen der Reste X entstehen. Besonders günstige Wirkungen besitzen die an beiden Asymmetriezentren des 1,2-Diphenyl-ethylendiamin-Teils gleich konfigurierten Komplexe.

Hinsichtlich der Geometrie am Platinatom handelt es sich bei den erfindungsgemäßen Verbindungen der Formel I stets um die cis-Verbindungen.

Das Ausgangsamin II wird beispielsweise als Racemat, als Meso-Verbindung, als reine rechtsbeziehungsweise linksdrehende Form oder in einer sonstigen diastereomeren Form eingesetzt. Diese Konfiguration bleibt bei der Herstellung des Platinkomplexes erhalten.

Das Verfahren zur Herstellung der erfindungsgemäßen Verbindungen I wird in einem Lösungsmittel bei Temperaturen zwischen 0 und 90°C, vorzugsweise 10 bis 60°C, insbesondere 20 bis 50°C durchgeführt. Als Lösungsmittel kommen beispielsweise in Frage: Wasser, $C_1$—$C_6$-Alkanole (Methanol, Ethanol, tert.-Butanol), Tetrahydrofuran, Dioxan, Dimethylsulfoxid, Dimethylformamid, Ethylenglykoldimethylether, Diethylenglykoldimethylether sowie Gemische dieser Lösungsmittel, insbesondere Mischungen mit Wasser.

Die beiden Reaktionskomponenten (Platin-Verbindung und Verbindung II) werden vorzugsweise in äquimolaren Mengen eingesetzt. Der pH-Wert der Reaktionslösung soll zwischen 5 und 7, vorzugsweise bei pH 6 liegen. Die Einstellung des pH-Wertes erfolgt insbesondere durch Zusatz von Alkali, vorzugsweise wäßriger Natronlauge oder Kalilauge oder beispielsweise auch mittels Natriumcarbonat.

Als Tetrahalogen-platin(II)-Verbindungen (Säure sowie Komplexsalze) kommen die entsprechenden Tetrachloro-, Tetrabromo- und Tetrajodo-Verbindungen in Frage. Falls Platin(II)-halogenide als Ausgangskomponente eingesetzt werden, kommen die gleichen Halogenatome in Frage.

Als einwertige Kationen kommen in Betracht: Alkali-Ionen, insbesondere Natrium und Kalium; es können aber auch die Lithium, Rubidium, Cäsium, oder Thalliumsalze verwendet werden, ebenso $NH_4^+$, $NR_4^+$, $PR_4^+$ oder $AsR_4^+$, in denen R ein $C_1$—$C_6$-Alkylrest oder ein Phenylrest ist. Zweiwertige Kationen können sein: Erdalkali-Ionen, insbesondere $Mg^{2+}$ und $Ca^{2+}$, aber auch $Zn^{2+}$. Als Platin(II)-halogenide kommen beispielsweise $PtCl_2$, $PtBr_2$ und $PtJ_2$ in Frage.

Die Verbindung II wird entweder in Form des Diamins oder in Form eines Säureadditionssalzes eingesetzt: zum Beispiel als Monohydrochlorid oder Dihydrochlorid, Mono- oder Dihydrobromid, Mono- oder Dihydrojodid oder als Salz mit einer anderen üblichen Säure. Insbesondere kommen auch die Säuren in Frage, deren Anionen die Reste X bilden. Weiterhin kann das Diamin in Form des Acetats beziehungsweise Diacetats eingesetzt werden, wobei gegebenenfalls vor dem Mischen der Reaktionskomponenten Kaliumchlorid (beispielsweise 2 Mol pro 1 Mol Verbindung II) zugesetzt wird. Ebenso kann das Diamin II in Form des Carbonats eingesetzt werden.

In Verbindungen der Formel I können vorhandene freie phenolische Hydroxygruppen durch $C_1$—$C_6$-Alkanoylgruppen acyliert werden. Diese Alkanoylgruppen können Halogenatome (zum Beispiel Cl, Br) oder $C_1$—$C_4$-Alkansulfonyloxygruppen enthalten. Diese Acylierung kann beispielsweise mittels $C_1$—$C_6$-Alkanoylhalogeniden oder den Anhydriden der $C_1$—$C_6$-Carbonsäuren bei Temperaturen zwischen 10 und 80°C, insbesondere 20—30°C in Anwesenheit üblicher säurebindender Stoffe erfolgen. Die Alkanoylhalogenide beziehungsweise Anhydride können gegebenenfalls mindestens 1 Halogenatom oder mindestens eine $C_1$—$C_4$-Alkansulfonyloxygruppe enthalten. Insbesondere kommen als säurebindende Stoffe aliphatische tertiäre Amine wie zum Beispiel Diisopropylethylamin in Betracht. Als inerte Lösungs- beziehungsweise Suspensionsmittel für die Acylierung kommen beispielsweise in Frage: niedere aliphatische Halogenkohlenwasserstoffe (Chloroform), aprotische Lösungsmittel wie Amide, $C_1$—$C_4$-Alkylamide und $C_1$—$C_4$-Dialkylamide aliphatischer $C_1$—$C_4$-Carbonsäuren (Dimethylformamid, Dimethylacetamid), N-Methyl-pyrrolidon, Dimethylsulfoxid oder Mischungen dieser Mittel.

Man kann diese Acylierung zum Beispiel aber auch in einem Zweiphasensystem beispielsweise Wasser/Chloroform durchführen, wobei sich dann der acylierte Platin(II)-Komplex im allgemeinen in der Wasserphase und das Gemisch von Säurechlorid und tertiärem Amin (Diisopropylethylamin) in der

Chloroformphase befindet. Der acylierte Platin(II)-Komplex läßt sich durch Zugabe eines entsprechenden Anions in einen weniger wasserlöslichen, gewünschten Komplex überführen und kann durch Abfiltrieren isoliert werden. Insbesondere gilt dies, wenn in der Wasserphase der acylierte Dihydroxo-platin(II)-Komplex vorliegt. Überhaupt ist es zweckmäßig, bei der Acylierung in einem Zweiphasensystem das Anion X des Platinkomplexes der Formel I vor der Acylierung mittels eines Anionenaustauschers gegen $OH^-$ auszutauschen, da der entsprechende Dihydroxoplatin(II)-Komplex gut wasserlöslich ist. Als Säurehalogenide kommen vorzugsweise die entsprechenden Chloride, Bromide und gegebenenfalls Jodide in Betracht. Als Anhydride von $C_1$—$C_6$-Carbonsäuren kommen insbesondere die symmetrischen Säureanhydride in Frage.

Der Austausch der Liganden X gegen andere Liganden kann beispielsweise mittels der Silberhalogenidfällung erfolgen. Hierzu wird beispielsweise eine Dihalogeno-(1,2-diphenyl-ethylendiamin)-platin(II)-Verbindung der Formel I, worin X Halogen (Chlor, Brom oder Jod) bedeutet in einem Lösungs- oder Suspensionsmittel bei Temperaturen zwischen 0 bis 90°C, vorzugsweise 10 bis 50°C, insbesondere 15 bis 25°C mit den Silbersalzen einer anderen Säure, die der Bedeutung X entspricht, umgesetzt. Man kann hierbei aber auch als Silbersalz Silbernitrat (zum Beispiel wäßrige Silbernitrat-Lösung) verwenden und erhält einen ionischen Diaquokomplex der Formel

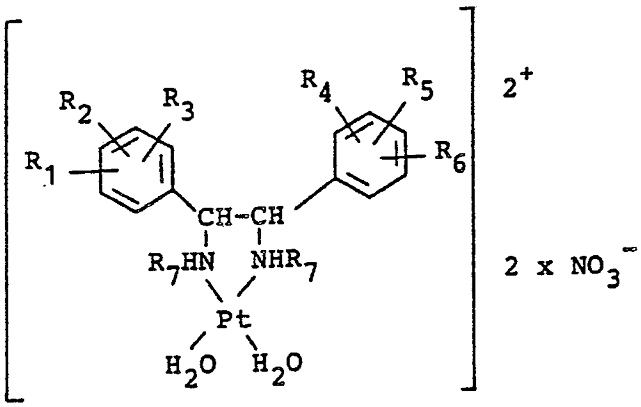

Aus diesem Komplex läßt sich der schwach gebundene Ligand Wasser leicht durch affinere Anionen (zum Beispiel $Cl^-$, $Br^-$ in Form von NaCl, KCl, NaBr, KBr, Malonat$^{2-}$, Chloracetat$^{(-)}$, Oxalat$^{2-}$, 1,1-Cyclobutandicarbonsäure-Anion$^{2-}$ sowie die übrigen angegebenen Säurereste X verdrängen, angewandt in Form der Säuren oder ihrer Salze, insbesondere ihrer Alkalisalze.

Die gleichen Verbindungen lassen sich auch durch Umsetzung äquimolarer Mengen von HX und Nitrat-freiem Platinkomplex (letzterer unter Verwendung von Anionenaustauschern in der Hydroxidform, zum Beispiel Dowex 1—8X) gewinnen.

Ein Austausch der Abgangsgruppe (zum Beispiel $SO_4^{2-}$-beziehungsweise Oxalatanion$^{2-}$) ist im Falle der Sulfato- beziehungsweise Oxalato-(1,2-diphenyl-ethylendiamin)-platin(II)-Verbindungen auch durch Umsetzung mit Erdalkalisalzen, die den gewünschten X-Liganden (zum Beispiel Glycerinsäure) enthalten, möglich, sofern der entstehende Komplex wasserlöslich ist und damit die Abtrennung des schwer wasserlöslichen Erdalkalisulfats oder -oxalats erlaubt.

Für dieses Verfahren geeignete X-Liganden sind vorzugsweise die Anionen von Hydroxycarbonsäuren, Sulfonsäuren, Halogenessigsäuren, Salpetersäure.

Die Lösungs- beziehungsweise Suspensionsmittel, die für das Herstellungsverfahren der Verbindungen I angegeben wurden, kommen auch für die Austauschreaktion in Frage (insbesondere eignen sich Wasser und Dimethylformamid sowie ferner Methanol, Ethanol, tert.-Butanol). Die Austauschreaktion wird beispielsweise in einem pH-Bereich zwischen 3 und 7 durchgeführt.

Herstellung von nicht bekannten Ausgangsaminen der Formel II:

Herstellung von 1,2-Diaryl-ethylendiaminen mit symmetrisch substituierten Phenylresten der Formel II, soweit diese nicht beschrieben sind.

Hierzu wird auch auf folgende Literaturstellen verwiesen: Journal of Medicinal Chemistry, 1982, Band 25, Seite 1374—1377; Chemische Berichte 1976, Band 109, Seite 1.

A. Allgemeine Vorschrift der Etherspaltung mit $BBr_3$ zur Darstellung von hydroxysubstituierten meso- und d,l-1,2-Diarylethylendiaminen der Formel II

5,5 mmol des betreffenden methoxysubstituierten mesobeziehungsweise d,l-1,2-Diarylethylendiamins werden in 150 ml wasserfreiem $CH_2Cl_2$ gelöst und auf −50°C abgekühlt. Unter Stickstoffatmosphäre werden 22 mmol Bortribromid hinzugegeben und 30 Minuten bei dieser Temperatur gerührt. Nach weiteren 18 Stunden Rühren bei Raumtemperatur wird das überschüssige $BBr_3$ unter Eiskühlung vorsichtig mit Methanol hydrolysiert und das Lösungsmittelgemisch abrotiert. Der Rückstand wird in 50 ml Wasser aufgenommen und der pH-Wert mit gesättigter $NaHCO_3$-Lösung auf pH 7 eingesetellt. Man saugt den Niederschlag ab, wäscht mit Wasser, Ethanol und $CH_2Cl_2$ und trocknet über $P_2O_5$. In der Regel erhält man

analysenreine Produkte. Beispielsweise werden die Ausgangsamine für die Beispiele 6, 8, 12, 13 und 16 auf diese Weise erhalten.

Die Etherspaltung kann zum Beispiel auch mit Bromwasserstoffsäure gemäß folgender Vorschrift erfolgen: 0,01 Mol des betreffenden methoxysubstituierten meso-1,2-Diarylethylendiamins werden in 60 ml 47 %iger HBr 24—48 Stunden unter Rückfluß erhitzt. Nach Abkühlen wird der Niederschlag abgesaugt, mit sehr wenig Eiswasser gewaschen und in Wasser gelöst. Man versetzt mit so viel 20 %iger NaOH-Lösung, bis das ausgefallene Ethylendiamin als Phenolat wieder in Lösung geht. Nach Filtrieren wird mit 2 N HCl auf pH 7 eingestellt und der Niederschlag abgesaugt. Man wäscht mit Wasser und Ethanol und trocknet das meist analysenreine Produkt über $P_2O_5$. Auf diese Weise wurden zum Beispiel die Ausgangsamine für die Beispiele 2, 4 und 10 erhalten.

B. Allgemeine Vorschrift zur Darstellung der mesokonfigurierten 1,2-Diarylethylendiamine der Formel II über die meso→meso-Diaza-Cope-Umlagerung

0,5 Mol N,N'-Disalicyliden-meso-1,2-diarylethylendiamin werden in 200 ml 2 N $H_2SO_4$ beziehungsweise halbkonzentrierter $H_2SO_4$ suspendiert. Der bei der Hydrolyse frei werdende Salicylaldehyd wird mittels Wasserdampfdestillation aus der Reaktionsmischung entfernt. Ist im Destillat kein Aldehyd mehr nachzuweisen, wird die Reaktionslösung heiß filtriert und nach Abkühlen vorsichtig mit 20 %iger NaOH-Lösung auf pH 11—12 eingestellt. Der Niederschlag wird mit $CH_2Cl_2$ extrahiert, die vereinigten Extrakte werden mit Wasser gewaschen und über $MgSO_4$ getrocknet. Das Lösungsmittel wird so weit eingeengt, bis das Produkt auszufallen beginnt. Man gibt etwa 100 ml Ether hinzu und läßt im Eisschrank kristallisieren. Das Ethylendiamin wird abgesaugt, mit Ether gewaschen und in Ethanol umkristallisiert.

Die Herstellung der entsprechenden N,N'-Disalicylidenmeso-1,2-diarylethylendiamine (Diaza-Cope-Umlagerung) kann beispielsweise nach folgender Methode erfolgen: meso-1,2-Bis-(2-hydroxyphenyl)-ethylendiamin wird mit 2 Moläquivalenten des entsprechenden Benzaldehyds der Formel

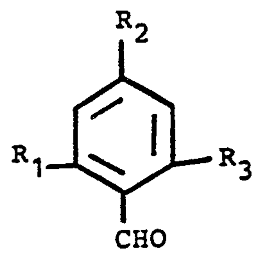

in Acetonitril unter Rückfluß erhitzt. Die Suspension wird ständig gerührt und so lange zum Sieden erhitzt, bis sich einheitliche, gelbe Kuben bilden (ca. 4—5 Stunden). Nach Abdestillieren der Hälfte des Lösungsmittels und Abkühlen auf Raumtemperatur wird der Niederschlag abgesaugt, mit wenig Acetonitril gewaschen und im Vakuumexsikkator über $P_2O_5$ getrocknet. Für die Beispiele 15 und 16 wird die entsprechende Mischung in Toluol 24 Stunden am Wasserabscheider unter Rückfluß erhitzt. Es kann nur ein Gemisch aus N,N'-Disalicyliden-meso-1,2-bis(2,6-dimethyl-4-methoxyphenyl)ethylendiamin und dem nicht umgelagerten Diimin N,N'-bis(2,6-dimethyl-4-methoxybenzyliden)-meso-1,2-bis(2-hydroxyphenyl)ethylendiamin isoliert werden, das ohne weitere Trennung weiter umgesetzt wird.

C. Allgemeine Vorschrift zur Synthese d,l-konfigurierter 1,2-Diarylethylendiamine der Formel II über die entartete Diaza-Cope-Umlagerung

0,1 Mol des betreffenden N,N'-Dibenzyliden-meso-1,2-diarylethylendiamins werden in einem 1 Liter-Dreihalskolben unter Rühren im Ölbad so lange auf dessen Schmelztemperatur gehalten, bis die Kristallmasse vollständig geschmolzen ist. Nach Abkühlen auf ca. 80°C werden 250 ml 2 N $H_2SO_4$ zugegeben und die Suspension wird einer Wasserdampfdestillation unterzogen, bis kein Aldehyd mehr übergeht. Die klare Lösung wird heiß filtriert und man läßt sie über Nacht zur Kristallisation bei Raumtemperatur steht. Das kristalline Dihydrosulfat des racemischen 1,2-Diarylethylendiamins wird abfiltriert und mit wenig eiskalter 2 N $H_2SO_4$ nachgewaschen. Anschließend wird es im Scheidetrichter mit 2 N NaOH-Lösung überschichtet und mit $CH_2Cl_2$ extrahiert. Nach trocknung über $MgSO_4$ rotiert man das Lösungsmittel ab und kristallisiert den öligen Rückstand in Ethanol um. Bei schwer kristallisierbaren Produkten kann man das Dihydrochlorid mit etherischer HCl ausfällen. Das Filtrat enthält das Dihydrosulfat des meso-konfigurerten 1,2-Diarylethylendiamins, das analog Methode B zurück-isoliert werden kann.

Die hierbei verwendeten N,N'-Dibenzyliden-meso-1,2-diarylethylendiamine können zum Beispiel wie folgt erhalten werden:

Das jeweilige meso-1,2-Diarylethylendiamin wird in Acetonitril gelöst und nach zugabe von 2 Moläquivalenten des entsprechenden, wie unter B angegebenen Benzaldehyds, 3 Stunden unter Rückfluß erhitzt. Man läßt abkühlen, saugt auf einem Büchnertrichter ab und wäscht mit etwas Acetonitril und Aceton nach. Trocknung im Exsikkator über $P_2O_5$.

D. Allgemeine Vorschrift zur Darstellung d,l-konfigurierter 1,2-Diarylethylendiamine über die d,l→d,l-Diaza-Cope-Umlagerung

25 mmol d,l-1,2-Bis(4-methoxyphenyl)ethylendiamin werden in 250 ml Methanol gelöst und zu 50 mmol des entsprechenden, wie unter B angegebenen, Benzaldehyds, gelöst in Methanol, gegeben. Die Lösung wird 24 Stunden unter Rückfluß erhitzt und anschließend das Lösungsmittel abrotiert. Der ölige Rückstand kann säulenchromatographisch über Kieselgel 60 aufgetrennt werden. Das aufgetragene Diimingemisch hydrolysiert meistens bereits auf der Säule und man kann durch geeignete Wahl und Abfolge von Lösungsmittelgemischen isomerenfreies d,l-Produkt isolieren. Nach Zusatz von etherischer HCl zur ethanolischen Lösung kann das d,l-1,2-Diarylethylendiamin kristallin als Dihydrochlorid erhalten werden. Nach dieser Methode wurde zum Beispiel das Ausgangsamin für die Verbindung gemäß Beispiel 11 erhalten.

E. Allgemeine Vorschrift zur Darstellung N,N'-dialkylierter 1,2-Diarylethylendiamine durch reduktive Dimerisierung der entsprechenden Benzaldehydalkalimine mit Tetraphenylethandiol

Eine Mischung von 0,04 Mol Benzaldehydalkylamin der Formel

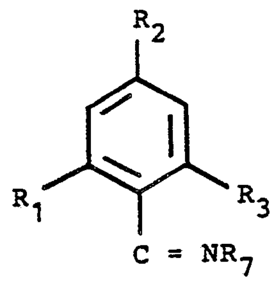

und 0,04 Mol 1,1,2,2-Tetraphenylethandiol im 200 ml 2-Propanol wird 24 Stunden unter Rückfluß erhitzt. Nach Abkühlen wird der Niederschlag (meso-Isomeres) abgesaugt, in $CH_2Cl_2$ gelöst und mit 2 N HCl extrahiert. Die wässrige Phase wird mit 2 N NaOH alkalisiert und mit $Ch_2Cl_2$ extrahiert. Die organische Phase wird mit Wasser gewaschen und über $MgSO_4$ getrocknet. Nach Abrotieren des Lösungsmittels erhält man das meso-Produkt in kristalliner Form, das in Ethanol umkristallisiert wird. Das Filtrat des Reaktionsgemisches wird einrotiert und das verbleibende braune Öl über Kieselgel 60 säulenchromatographiert. Das isomerenfreie d,l-Produkt wird durch sukzessive Eluierung mit $CH_2Cl_2$, Ether und Methanol erhalten und kann in Acetonitril umkristallisiert werden.

Die Herstellung der Benzaldehydalkalimine erfolgt aus den entsprechend substituierten Benzaldehyden und dem Alkylamin $NH_2R_7$ in der üblichen Weise, beispielsweise in Chloroform, zuerst bei Raumtemperatur (2 Stunden Rühren) und anschließendem Erhitzen unter Rückfluß (30 Minuten).

Im folgenden werden die Herstellungsvorschriften für verschiedene noch nicht beschriebene Benzaldehyde angegeben, die bei den zuvor angegebenen Methoden zur Herstellung der Amine II als Ausgangsaldehyde eingesetzt werden. Andere nicht bekannte Benzaldehyde können in analoger Weise erhalten werden.

2-Jod-4-methoxy-benzaldehyd

0,1 Mol 4-Methoxybenzaldehyddimethylacetal wird in einem flammengetrockneten Kolben unter $N_2$-Atmosphäre mit 500 ml wasserfreiem Ether versetzt und auf −60°C im Trockeneis-Aceton-Kältebad abgekühlt. Mit Hilfe einer langen, biegsamen Stahlnadel werden 0,11 Mol einer Lösung von tertiärem Butyllithium in Hexan durch eine Serumkappe unter Stickstoffdruck aus der Vorratsflasche übergetrieben. Die Farbe ändert sich schlagartig nach gelb. Man läßt die Temperatur auf −25°C steigen und rührt noch 3 Stunden, bis sich ein gelber, voluminöser Niederschlag gebildet hat. Das so erhaltene 2-Lithium-4-methoxybenzaldehyddimethylacetal wird sofort tropfenweise mit 0,11 Mol $J_2$, gelöst in 200 ml wasserfreiem Ether, bei −25°C versetzt. Die bleibende Jodfarbe zeigt das Ende der Reaktion an. Die Lösung wird noch 30 Minuten bei Raumtemperatur gerührt und anschließend mit Wasser versetzt. Man extrahiert mit Ether, wäscht sukzessive mit wässriger $Na_2SO_3$-Lösung, 2 N HCl-Lösung, gesättigter $NaHCO_3$-Lösung und Wasser und trocknet über $MgSO_4$. Das Lösungsmittel wird abrotiert und der Rückstand in Methanol umkristallisiert.

farblose Nadeln, F. 109—111°C

2-Brom-4-methoxybenzaldehyd

2-Lithium-4-methoxybenzaldehyddimethylacetal wird schnell mit 0,11 Mol Brom bei −60°C versetzt. Anschließend wird 1 Stunde bei Raumtemperatur gerührt. Man versetzt mit Wasser, extrahiert mit Ether und wäscht sukzessive mit wässriger $NaSO_3$-Lösung, 2 N HCl-Lösung, gesättigter $NaHCO_3$-Lösung und Wasser. Nach Trocknen über $MgSO_4$ und Abrotieren des Ethers bleibt ein rotbraunes Öl zurück, das einer Wasserdampfdestillation unterzogen wird. Das Destillat extrahiert man mit $CH_2Cl_2$, trocknet über $MgSO_4$

und engt ein. Der Rückstand wird über Kieselgel 60 (Fa. Merck, Darmstadt) mit Ether/Petrolether 40—60 1:2 säulenchromatographiert ($R_f$~0,5—0,6).

farblose, lange nadeln, F. 73—74°C.

2-Fluor-4-methoxy-benzaldehyd

Durch Umsetzung von 3-Fluoranisol mit Brom in Gegenwart von Eisenpulver in $CHCl_3$ bei −60°C im Trockeneis-Acetonkältebad analog der von Quelet, R.; Paty M. in Procés Verbaux soc. sci. phys. et nat. Bordeaux, 1944—45, 19 angegebenen Methode erhält man nach fraktionierter Destillation am Wasserstrahlvakuum ein Isomeren-Gemisch aus 4-Brom-3-fluoranisol und 2-Brom-5-fluoranisol, das ohne Trennung weiterverarbeitet wird.

farblose Flussigkeit, Siedepunkt 93—95° C/14 Torr

0,1 Mol n-Butyllithium-Hexanlösung wird in Stickstoffatmosphäre mit 150 ml wasserfreiem Ether versetzt und auf −70°C abgekühlt. 0,1 Mol des zuvor beschriebenen Isomerengemisches wird in 50 ml wasserfreiem Ether aufgenommen und mit solcher Geschwindigkeit zugetropft, daß die Reaktionstemperatur −55°C nicht überschreitet. Man rührt bei dieser Temperatur 15 Minuten weiter und setzt dann N-Formylpiperidin, gelöst in 25 ml wasserfreiem Ether, zu, wobei die Temperatur wiederum unter −55°C gehalten wird. Fällt der anschließende Test auf metallorganische Verbindung mit Michler's Keton negativ aus, wird auf Raumtemperatur gebracht, mit 2 N HCl angesäuert und die organische Phase abgetrennt. Die Etherphase wäscht man mit gesättigter $NaHCO_3$-Lösung und Wasser, trocknet über $MgSO_4$ und zieht das Lösungsmittel ab. Der ölige Rückstand wird über Kieselgel 60 mit $CH_2Cl_2$/Petrolether 40—60 1:1 chromatographiert ($R_f$~0,45—0,5).

farblose Nadeln, F. 42,5—44°C

Analog wird der 4-Methoxy-2-trifluormethylbenzaldehyd (F. 38—39°C, nach Reinigung durch Säulenchromatographie über Kieselgel mit Ether/Petrolether) über ein Gemisch aus 4-Brom-3-trifluormethylanisol/2-Brom-5-trifluormethylanisol (Siedepunkt 46—47°C/0,6 Torr) erhalten. Durchführung der Bromierung: 18 Stunden bei Raumtemperatur.

2,6-Dichlor-4-methoxybenzaldehyd

1 Mol gefällter, aktiver Braunstein und 800 ml Benzol werden 2 Stunden am Wasserabscheider zum Sieden erhitzt. Anschließend werden 0,2 Mol 2,6-Dichlor-4-methoxybenzyalkohol zugegeben und über Nacht wiederum am Wasserabscheider unter Rückfluß erhitzt. Nach Abkühlen auf Raumtemperatur wird der Braunstein über einen Glasfiltertiegel abgesaugt und mit Benzol nachgewaschen. Nach Abrotieren des Lösungsmittels erhält man zumeist ein sehr reines Produkt, das nach Bedarf, jedoch unter größerem Ausbeuteverlust, aus Methanol umkristallisiert werden kann.

farblose Nadeln, F. 108—111°C

Der eingesetzte 2,6-Dichlor-4-methoxybenzylalkohol wird die folgt erhalten:

0,56 Mol 3,5-Dichloranisol werden mit 30,3 g Paraformaldehyd in 1,5 Liter konzentrierer HCl und 15 ml konzentrierter $H_2SO_4$ 7 Stunden bei 60°C Reaktionstemperatur gerührt. Nach Abkühlen werden die Phasen getrennt, die wässrige Phase mit $CH_2Cl_2$ extrahiert und die vereinigten organischen Phasen mit Wasser gewaschen. Nach Trocknen über $MgSO_4$ und Abrotieren des Lösungsmittels bleibt ein farbloses Öl zurück. Zu diesem Öl werden 530 ml 2 N NaOH-Lösung, 530 ml Wasser und 530 ml Dioxan gegeben und die Mischung 3 Stunden zum Sieden erhitzt. Die organische Phase wird nach Abkühlen abgetrennt, die wässrige Phase wird mit $CH_2Cl_2$ ausgeschüttelt und anschließend werden die vereinigten organischen Phasen mit Wasser gewaschen und über $MgSO_4$ getrocknet. Nach Abrotieren des Lösungsmittels wird der ölige Rückstand mit 100 ml $CHCl_3$ versetzt und der sich bildende Niederschlag abfiltriert. Man rotiert das Chloroform ab und trennt den öligen Rückstand säulenchromatographisch über Kieselgel 60 mit einem 1:1 Gemisch aus Petrolether 40—60/Ether auf.

farblose Nadeln, F. 78—79°C

2-Chlor-4-methoxybenzaldehyd

0,1 Mol 2-Lithium-4-methoxybenzaldehyddimethylacetal wird bei −60°C mit 0,11 Mol Benzolsulfonsäurechlorid, gelöst in 50 ml wasserfreiem Ether, mittels einer Spritze zugegeben. Man läßt anschließend 1 Stunde bei Raumtemperatur rühren. Durch Zusatz von Eiswasser löst sich der gebildete weiße Niederschlag wieder auf. Man extrahiert mit Ether, wäscht mit 2 N HCl-Lösung, gesättigter $NaHCO_3$-Lösung und Wasser, trocknet über $MgSO_4$ und zieht das Lösungsmittel ab. Der Rückstand wird über Kieselgel 60 (Firma Merck, Darmstadt) mit Ether/Petrolether 40—60 1:2 säulenchromatographisch aufgetrennt.

$R_f$ 0,75—0,8

farblose Nadeln, F. 60—61°C.

Die erfindungsgemäßen Verbindungen zeigen an der menschlichen MDA—MB 231-Brustkrebszellinie (Dissertation Jennerwein, Universität Regensburg, 1985, Seite 151) an der Leukämie P 388 (Maus) (siehe Dissertation Johann F.-X. Karl, Universität Regensburg, 1985, Seiten 88—89; 93, 94; 99—102), am DMBAinduzierten Mammatumor der Ratte und an der MCF 7-Brustkrebszellinie des Menschen eine gute Antitumor-Wirkung.

Beispielsweise wird am Mammacarcinom der Ratte, welches durch 7,12-Dimethylbenz[a]-anthracen (DMBA) induziert wurde, mit der Verbindung gemäß Beispiel 18 bei einer Dosis von 3 × 6,5 mg/kg Körpergewicht/Woche bei 88% der Tumore ein kompletter Rückgang festgestellt. Bei der Testung an der hormonunabhängigen menschlichen Mammatumorzellinie MDA—MB 231 zeigen die erfindungsgemäßen Verbindungen in vitro beispielsweise in Konzentrationen zwischen $10^{-5}$ und $10^{-7}$ Mol/Liter eine 50 %ige Wachstumshemmung an dieser Zellinie. In derselben Größenordnung liegt die Hemmung des [$^3$H]-Thymidneinbaus. An der lymphocytischen Leukämie P 388 (Maus) wirken die erfindungsgemäßen Verbindungen zum Beispiel in Dosierungen zwischen 10 und 50 mg/kg Maus (intraperitoneale Applikation).

Die Wirkungsrichtung der erfindungsgemäßen Verbindungen ist mit derjenigen des bekannten Arnzeimittels Cisplatin vergleichbar.

Die niedrigste, bereits zu 65% kompletten Remissionen führende Dosis in dem obenangegebenen Tierversuch ist

3 × 3,25 mg/kg subkutan, beziehungsweise auch intravenös.

Als allgemeiner Dosisbereich für die Wirkung (Tierversuch wie oben) kommt beispielsweise in Frage:
5—20 mg/kg oral, insbesondere 10 mg,
1—10 mg/kg intravenös, insbesondere 6,5 mg.

Indikationen, für die die erfindungsgemäßen Verbindungen in Betracht kommen können: Tumorerkrankungen, besonders Mamma-, Endometrium-, Cervix-, Ovarial- und Prostatacarcinom, ferner Leukämien, Hodenteratom und Blasencarcinom.

Die pharmazeutischen Zubereitungen enthalten im allgemeinen zwischen 100 bis 200, vorzugsweise 150 mg der erfindungsgemäßen aktiven Komponente.

Die Verabreichung kann beispielsweise in Form von Tabletten, Kapseln, Pillen, Dragees, Zäpfchen, Salben, Gelees, Cremes, Puder, Stäubepulver, Aerosolen oder in flüssiger Form erfolgen. Als flüssige Anwendungsformen kommen zum Beispiel in Frage: Ölige oder alkoholische beziehungsweise wässrige Lösungen sowie Suspensionen und Emulsionen. Bevorzugte Anwendungsformen sind Tabletten, die zwischen 100 und 200 mg oder Lösungen, die zwischen 0,02 bis 0,04% an aktiver Substanz enthalten.

Die Einzeldosis der erfindungsgemäßen aktiven Komponenten kann beispielsweise liegen

a) bei oralen Arzneiformen zwischen 100 und 200 mg, vorzugsweise 150 mg,

b) bei parenteralen Arzneiformen (zum Beispiel intravenös, intramuskulär) zwischen 100 und 200 mg/$m^2$, vorzugsweise 150 mg/$m^2$ Körperoberfläche,

c) bei Arzneiformen zur rektalen oder vaginalen Applikation zwischen 1 und 5%, vorzugsweise 2,5%.

d) bei Arzneiformen zur lokalen Applikation auf die Haut und Schleimhäute (zum Beispiel in Form von Lösungen, Lotionen, Emulsionen, Salben und so weiter) zwischen 1 und 5%, vorzugsweise 2,5%.

(Die Dosen sind jeweils bezogen auf die freie Base)

Beispielsweise können 3 mal täglich 1 bis 4 Tabletten mit einem Gehalt von 100 bis 200 mg wirksamer Substanz oder zum Beispiel bei intravenöser Injection 1000 ml mit einem Wirkstoffgehalt entsprechend 100 bis 200 mg/$m^2$ Körperoberfläche empfohlen werden. Bei oraler Verabreichung ist die minimale tägliche Dosis beispielsweise 300, die maximale tägliche Dosis bei oraler Verabreichung soll nicht über 800 mg liegen.

Die akute Toxizität der erfindungsgemäßen Verbindungen an der Maus (ausgedrückt durch die LD 50/kg; Methode nach Miller und Tainter: Proc. Soc. Exper. Biol. a. Med. *57* (1944) 261) liegt beispielsweise bei oraler Applikation oberhalb 100 mg/kg intraperitoneal, häufig oberhalb 1000 mg/kg.

Die Arzneimittel können in der Humanmedizin, der Veterinärmedizin allein oder im Gemisch mit anderen pharmakologisch aktiven Stoffen verwendet werden.

## BEISPIELE

Die Beispiele 1—16 sind in der Tabelle 1 aufgeführt. Die physikalischen Kenndaten der Platin(II)-Komplexe sin in der Tabelle 2 angegeben.

Die Beispiele 1—16 betreffen die Herstellung von erfindungsgemäßen Platin(II)-Komplexen der folgenden Formel:

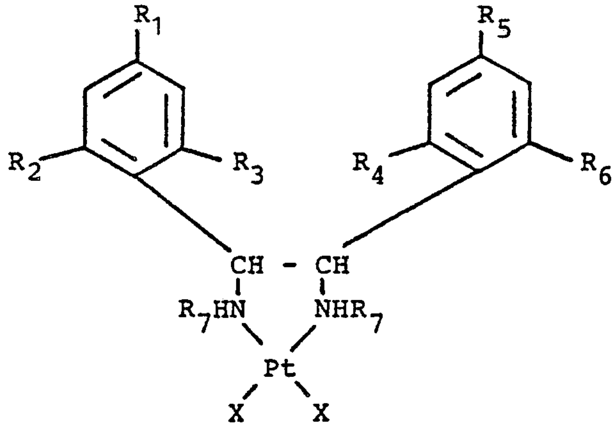

Sämtliche Komplexe gemäß den Beispielen 1—16 stellen blaßgelbe bis gelbe Pulver dar.

Allgemeine Vorschrift zur Darstellung der Platinkomplexe im wässrigen Medium:

1 mmol, 1,2-Diarylethylendiamin wird in 20 ml destilliertem $H_2o$ suspendiert und durch Zusatz von ml 2 N HCl gelöst. Die filtrierte Lösung wird mit 20 ml tertiärem Butanol versetzt und auf 40°C erwärmt. Unter Rühren wird langasam so viel 0,5 N NaOH zugetropft, bis die freie Base auszufallen beginnt (pH notieren). Der Lösung werden anschließend tropfenweise 415 mg (1 mmol) $K_2PtCl_4$, gelöst in 10 ml $H_2O$, zugesetzt. Man läßt unter Lichtausschluß bei 40°C rühren und stellt in Abständen von 4—5 Stunden den pH auf den notierten Wert nach. Ein über längere Zeit konstanter pH-Wert zeigt das Ende der Reaktion an. Nach Abkühlen auf Raumtemperatur wird der gelbe, meist analysenreine Niederschlag über eine Fritte (G—4) abgenutscht, sukzessive mit 2 N HCl und Wasser gewaschen und in der Trockenpistole bei 100°C über $P_2O_5$ getrocknet. Falls notwendig, werden die Komplexe zur Reinigung in wenig Dimethylformamid gelöst und mit 5% NaCl-Lösung gefällt.

Nach dieser Methode werden die Komplexe gemäß den Beispielen 1—13 sowie 15 und 16 hergestellt.

Allgemeine Vorschrift zur Darstellung der Platinkomplexe im organischen Medium:

1 mmol des betreffenden 1,2-Diarylethylendiamins werden in 20 ml Dimethylformamid gelöst beziehungsweise suspendiert und mit 1 mmol $K_2PtCl_4$, gelöst in 5 ml $H_2O$/Dimethylformamid (1:1-Gemisch) versetzt. Die Mischung wird im Dunkelen bei Raumtemperatur gerührt, bis ein Farbschlag von rot nach gelb erfolgt ist. Sollte sich nach 3 Tagen noch keine Farbänderung bemerkbar machen, dann wird 1 ml Dimethylsulfoxid zugegeben und die Lösung weitere 3—4 Stunden gerührt. Die gelbe Lösung wird anschließend am Hochvakuum bis zur Trockne eingeengt. Zum gelben, öligen Rückstand werden 50 ml 5%ige NaCl-Lösung gegeben und die Mischung 6 Stunden gerührt. Der gelbe, feinkristalline Niederschlag wird abgesaugt, mit 2 N HCl und Wasser gewaschen und über $P_2O_5$ in der Trockenpistole bei 100°C getrocknet.

Nach dieser Methode wird der Komplex gemäß Beispiel 14 hergestellt.

Tabelle 1

| Beispiel-Nr. | $R_2$, $R_4$ | $R_1$, $R_5$ | $R_3$, $R_6$ | $R_7$ (beide) | x | Konfiguration | Farbe |
|---|---|---|---|---|---|---|---|
| 1 | J | $OCH_3$ | H | H | Cl | meso | gelbes Pulver |
| 2 | J | OH | H | H | Cl | meso | hellgelbes Pulver |
| 3 | Br | $OCH_3$ | H | H | Cl | meso | hellgelbes Pulver |
| 4 | Br | OH | H | H | Cl | meso | hellgelbes Pulver |
| 5 | Cl | $OCH_3$ | H | H | Cl | meso | hellgelbes Pulver |
| 6 | Cl | OH | H | H | Cl | meso | hellgelbes Pulver |
| 7 | F | $OCH_3$ | H | H | Cl | meso | hellgelbes Pulver |
| 8 | F | OH | H | H | Cl | meso | hellgelbes Pulver |
| 9 | Cl | $OCH_3$ | Cl | H | Cl | meso | blaßgelbes Pulver |
| 10 | Cl | OH | Cl | H | Cl | meso | blaßgelbes Pulver |
| 11 | Cl | $OCH_3$ | Cl | H | Cl | d, ℓ | hellgelbes Pulver |
| 12 | Cl | OH | Cl | H | Cl | d, ℓ | blaßgelbes Pulver |
| 13 | Cl | $OCH_3$/OH* | Cl | H | Cl | meso | gelbes Pulver |
| 14 | Cl | $OCH_3$ | Cl | $CH_3$ | Cl | meso | blaßgelbes Pulver |
| 15 | Cl | $OCH_3$ | Cl | $CH_3$ | Cl | d, ℓ | blaßgelbes Pulver |
| 16 | Cl | OH | Cl | $CH_3$ | Cl | d, ℓ | blaßgelbes Pulver |

* der eine Rest $R_1$ ist OH, der andere ($R_5$) ist $OCH_3$

Tabelle 2

Physikalische Eigenschaften der Verbindungen von Tabelle 1:

IR = IR-Spektrum in KBr (br = breit, m = mittelstark, s = stark, sh = Schulter, vs = sehr stark, w = schwach)

$^1$H-NMR = Kernmagnetisches Resonanzspektrum, Verschiebung der Protonen in ppm
Aufnahme in deuteriertem Dimethylformamid ($d_7$ - DMF) / Tetramethylsilan bei 250 Megahertz
br = breit, s = Singulett, d = Dublett, t = Triplett, q = Quartett, m = Multiplett; J = Kopplungskonstante,
$J_{1,2}$ = zum Beispiel Kopplungskonstante für Protonen benachbarter C-Atome, $J_{HF}$ = zum Beispiel Kopplungskonstante die aus einer H··F-Kopplung resultiert

| Verbindung gemäß Beispiel | IR | $^1$H-NMR |
|---|---|---|
| 1 | 3250 m, 3180 m, 3090 m (ν-NH); 2960 w, 2940 w, (CH); 2830 w ($OCH_3$); 1600 s, 1580 s (δ-NH); 350 m, br (Pt-Cl) | 3,81 (s, 2 $OCH_3$); 4,54 (br, 2 NH); 5,58 (m, br, 2 NH); 6,50 (d, br, 2 CH); 6,96 (q, $J_{1,2}$=9 Hz, $J_{1,3}$=3 Hz, 2 Ar-H); 7,33 (d, $J_{1,3}$=3 Hz, 2 Ar-H); 8,25 (d, br, 2 Ar-H) |
| 2 | 3380 s, br (ν-OH); 3270 m, 3220 m, 3180 w, 3110 m (ν-NH); 1595 s, 1525 s, (δ-NH); 330 m, 320 m (Pt-Cl) | 4,48 (br, 2 NH); 5,46 (m, br, 2 NH); 6,45 (d, br, 2 CH); 6,82 (q, $J_{1,2}$=9 Hz, $J_{1-3}$=3 Hz, 2 Ar-H); 7,25 (d, $J_{1,3}$=3 Hz, 2 Ar-H); 8,14 (br, 2 Ar-H); 10,05 (br, 2 OH) |
| 3 | 3250 m, 3180 m, 3090 m (ν-NH); 2960 w, 2940 w (CH); 2830 w ($OCH_3$); 1605 s, 1565 s (δ-NH); 330 m, br (Pt-Cl) | 3,82 (s, 2 $OCH_3$); 4,72 (br, 2 NH); 5,61 (m, br 2 NH); 6,44 (d, br, 2 CH); 6,95 (q, $J_{1,2}$=9 Hz, $J_{1,3}$=3 Hz, 2 Ar-H); 7,08 (d, $J_{1,3}$=3 Hz, 2 Ar-H); 8,38 (d, br, $J_{1,2}$=7 Hz, 2 Ar-H) |
| 4 | 3450-3390 br (ν-OH); 3260 w, 3200 s, 3100 m (ν-NH) 1605 s, 1580 m (δ-NH); 325 w (Pt-Cl) | 4,67 (br, 2 NH); 5,52 (m, br, 2 NH); 6,38 (d, br, 2 CH); 6,80 (q, $J_{1,2}$=8 Hz, $J_{1,3}$=2 Hz, 2 Ar-H); 6,96 (d, $J_{1,3}$=2 Hz, 2 Ar-H); 8,26 (d, br, $J_{1,2}$=7 Hz, 2 Ar-H); 10,17 (br, 2 OH) |

Fortsetzung Tabelle 2

| Verbindung gemäß Beispiel | IR | |
|---|---|---|
| 5 | 3200 m, 3180 m, 3100 m (ν-NH); 2960 w (CH); 2830 w ($OCH_3$); 1605 s, 1570 s (δ-NH); 335 m, 325 m (Pt-Cl) | 3,82 (s, 2 $OCH_3$); 4,77 (br, 2 NH); 5,64 (m, br, 2 NH); 6,40 (d, br, 2 CH); 6,90 - 6,92 (m, 4 Ar-H); 8,41 (d, br, 2 Ar-H) |
| 6 | 3560 m; 3385 m (ν-OH); 3265 m, 3230 m, 3200 m, 3095 m (ν-NH); 1605 s, 1575 s (δ-NH); 320 w (Pt-Cl) | 4,71 (br, 2 NH); 5,54 (m, br, 2 NH); 6,34 (d, br, 2 CH); 6,74 - 6,78 (m, 4 Ar-H); 8,32 (d, br, 2 Ar-H); 10,20 (br, 2 OH) |
| 7 | 3250 m, 3170 m, 3100 m (ν-NH); 2960 w, 2940 w (CH); 2835 w ($OCH_3$); 1625 s, 1580 s (δ-NH); 330 m (Pt-Cl) | 3,80 (s, 2 $OCH_3$); 4,60 (br, 2 NH); 5,61 (m, br, 2 NH); 6,30 (d, br, 2 CH); 6,67 (q, $J_{1,3}$=3 Hz, $J_{HF1,2}$=12 Hz, 2 Ar-H); 6,77 (q, $J_{1,2}$=9 Hz, $J_{1,3}$=2 Hz, 2 Ar-H); 8,36 (t, $J_{1,2}$=9 Hz, $J_{HF1,3}$=9 Hz, 2 Ar-H) |
| 8 | 3400 s, br (ν-OH); 3260 s, 3210 s, 3105 m (ν-NH); 1625 s, 1595 s (δ-NH); 325 w (Pt-Cl) | 4,54 (br, 2 NH); 5,50 (m, br, 2 NH); 6,23 (d, br, 2 CH); 6,44 (q, $J_{1,3}$=2 Hz, $J_{HF1,2}$= 12 Hz, 2 Ar-H); 6,63 (q, $J_{1,2}$=9 Hz, $J_{1,3}$=2 Hz, 2 Ar-H); 8,23 (t, $J_{1,2}$=9 Hz, $J_{HF1,3}$=9 Hz, 2 Ar-H); 10,17 (br, 2 OH) |
| 9 | 3310 m, 3270 w, 3250 w, 3180 w, br, 3105 m (ν-NH); 3008 w, 2975 w, 2940 w (CH); 2835 w ($OCH_3$); 1602 s, 1555 s (δ-NH), 317 w (Pt-Cl) | 3,86 (s, 2 $OCH_3$); 5,35 (m, 2 $NH_2$); 6,75 (d, br, 2 CH); 7,01 (s, 2 Ar-H); 7,04 (s, 2 Ar-H) |
| 10 | 3370 s, br (ν-OH); 3295 m, 3245 m, 3160 m, br, 3085 w, 3060 w (ν-NH); 1607 s, 1560 s (δ-NH); 327 sh, 317 w (Pt-Cl) | 5,30 (m, 2 $NH_2$); 6,73 (d, br, 2 CH); 6,82 (s, 2 Ar-H); 6,85 (s, 2 Ar-H); 10,86 (s, 2 OH) |
| 11 | 3290 s, 3190 s, 3100 w, br (ν-NH); 2955 w, 2930 w (CH); 2830 ($OCH_3$); 1600 s, 1552 s (δ-NH); 335 m, 322 m (Pt-Cl) | 3,86 (s, 2 $OCH_3$); 4,99 (m, 2 NH); 5,58 (m, 2 NH); 6,90 (d, br 2 CH); 6,96 (d, J = 2 Hz, 2 Ar-H); 7,19 (d, J = 2 Hz, 2 Ar-H) |

Fortsetzung Tabelle 2

| Verbindung gemäß Beispiel | IR | $^1$H-NMR |
|---|---|---|
| 12 | 3240 s, br ($\nu$-OH); 3300 s ($\nu$-NH); 2880 w (CH); 1610 s, 1572 s ($\delta$-NH); 335 sh, 328 w (Pt-Cl) | 4,92 (m, 2 NH); 5,50 (m, 2 NH); 6,88 (d, br, 2 CH); 6,78 (d, J = 2 Hz, 2 Ar-H); 6,98 (d, J = 2 Hz, 2 Ar-H); 10,57 (s, 2 OH) |
| 13 | 3270 s, 3230 w, 3190 w ($\nu$-NH); 2960 w, 2940 w (CH); 2840 w ($OCH_3$); 1615 s, 1585 m, ($\delta$-NH); 325 w (Pt-Cl) | 3,86 (s, 1 $OCH_3$); 5,25-5,45 (m, br, 2 $NH_2$); 6,75 (br, 2 CH); 6,82 (s, 1 Ar-H); 6,85 (s, 1 Ar-H); 7,01 (s, 1 Ar-H); 7,04 (s, 1 Ar-H); 10,89 (s, 1 OH) |
| 14 | 3290 m, 3160 m, 3090 w ($\nu$-NH); 2960 w (CH); 1600 s, 1555 s ($\delta$-NH); 345 m, br (Pt-Cl) | 2,92 (s, 2 $CH_3$); 3,88 (s, 2 $OCH_3$); 5,04 (t, J = 2 Hz, 1 NH); 5,06 (t, J = 2 Hz, 1 NH); 6,05 (m, br, 2 CH); 7,06 (d, $J_{1,3}$= 3 Hz, 2 Ar-H); 7,13 (d, $J_{1,3}$=3 Hz, 2 Ar-H) |
| 15 | 3280 m, 3200 m, 3080 w ($\nu$-NH); 2990 w, 2940 m (CH); 1600 s, 1560 s ($\delta$-NH); 340 m, 335 sh (Pt-Cl) | 2,74 (s, 2 $CH_3$); 5,43 (t, J = 3 Hz, 1 NH); 5,45 (t, J = 3 Hz, 1 NH); 5,94 (m, br 2 CH); 7,04 (d, $J_{1,3}$=3 Hz, 2 Ar-H); 7,23 (d, $J_{1,3}$= 3 Hz, 2 Ar-$^{1,3}$H) |
| 16 | 3330 s, br ($\nu$-OH); 3230 m, 3190 m ($\nu$-NH); 2940 w (CH); 1610 s, 1570 s ($\delta$-NH); 340 m, 330 m (Pt-Cl) | 2,75 (s, 2 $CH_3$); 5,36 (t, J = 3 Hz, 1 NH) 5,39 (t, J = 3 Hz, 1 NH); 5,87 (d, br, 2 CH); 6,82 (d, $J_{1,3}$=3 Hz, 2 Ar-H); 7,00 (d, $J_{1,3}$= 3 Hz, 2 Ar-$^{1,3}$H); 10,85 (br, 2 OH) |

Beispiel 17

meso-Dichloro[1,2-bis-(2,6-dichlor-phenyl)-ethylendiamin]-platin-(II)

Herstellung erfolgt gemäß der angegebenen allgemeinen Vorschrift in wässrigem Medium. Die Substanz ist ein hellgelbes Pulver.

IR-Spektrum in KBr: 3375 w, 3315 m, 3280 w, 3225 m, 3200 w, 3060 w (νNH); 2960 w (CH); 1580 m, 1562 m (δNH); 325 sh, 317 w (Pt-Cl)

$^1H$—NMR: δ = 5,50 (m, 2 $NH_2$); 6.86 (d, br, 2 CH); 7,30—7,52 (m, 6 Ar-H)

Beispiel 18

d,l-Dichloro-[1,2-bis-(2,6-dichlor-phenyl)-ethylendiamin]-platin-(II)

Die Herstellung erfolgt gemäß der angegebenen allgemeinen Vorschrift in wässrigem Medium.

Die Substanz ist ein gelbes Pulver.

IR-Spektrum in KBr: 3300 s, 3195 m, br, 3100 w, 3060 w (νNH); 3000 w (CH); 1580 s, 1562 s (δNH); 330 sh, 322 m (Pt-Cl)

$^1H$—NMR: δ = 5,15 (m; 2 NH); 5,72 (m, br, 2 NH); 7,00 (d, br, 2 CH); 7,31—7,60 (m, 6 Ar-H)

Synthese von Pt-Komplexen gemäß den Beispielen 19—25.

Zur Lösung von 2 mmol des entsprechenden 1,2-Diphenylethylendiamin-dihydrochlorids in ca. 10 ml $H_2O$ werden 830 mg (2 mmol) $K_2PtCl_4$, gelöst in 8 ml $H_2O$ zugetropft. Die Lösung wird mit NaOH auf pH 5,5—6,5 gebracht. Man läßat unter Lichtausschluß bei Raumptemperatur rühren und neutralisiert die Lösung in Abständen von 1—2 Stunden. Die Konstanz des pH-Wertes zeigt das Ende der Reaktion an. Das Produkt wird abgesaugt mit $H_2O$ Chlorid-frei gewaschen und getrocknet.

Beispiel 19

(±)-Dichloro-[1,2-bis(4-fluor-phenyl)-ethylendiamin]-platin(II) ((±) 4—F)

Gelbes Pulver, F. ca. 380°C (Zersetzung)

IR-Spektrum in KBr: 3270 s, 3190 s ($NH_2$), 1610 s, 1515 s, 1240 s, 1170 m, 840 m, 320 w

$^1H$—NMR: δ =5,05 (breit, 2H, NH); 5,90 (br, 2H, NH); 6,50 (d, 2H, CH); 7,06 (t, $J_{HH}$ = 8,8 Hz, $J_{HF}$ = 8,9 Hz 4H, meta H); 7,74 (q, $J_{HH}$ = 8,8 Hz, $J_{HF}$ = 5,4 Hz, 4 H, ortho H).

Das Ausgangsamin wird zum Beispiel wie folgt hergestellt:

19,9 mmol 1-Azido-2-amino-1,2-bis-(4-fluor-phenyl)-ethan werden in ca. 60 ml absolutem Ether gelöst und unter Eiskühlung zu 1,52 g (40 mmol) $LiAlH_4$ in 60 ml absolutem Ether zugetropft, Nach 4,5 stündigem Rückflußerhitzen läßt man abkühlen und hydrolysiert bei 0—5°C mit feuchtem Ether und wenig Wasser. Vom Aluminiumhydroxid wird abgesaugt und dies mehrmals mit Methylenchlorid extrahiert. Nach Entfernen des Lösungsmittels bleibt das D,L-1,2-Bis(4-fluorphenyl)ethylendiamin als Öl zurück.

IR (Film/Base): 3380 m, 3300 m (NH), 1600 s, 1510 s, 1220 s, 830 s.

Das 1-Azido-2-amino-1,2-bis-(4-fluor-phenyl)-ethan wird aus 4,4'-Difluor-stilben über das Aziridin analog der in der DE—OS 34 05 611 (Seiten 17—19) angegebenen Vorschrift erhalten. Das hierbei als Zwischenprodukt erhaltene Carbamat wurde ohne weitere Reinigung weiterverarbeitet. Das 2,3-Bis(4-fluor-phenyl)-aziridin wurde durch Chromatographie an Kieselgel 60 mit Benzol/Methylenchlorid als Eluationsmittel gereinigt. Neben dem cis-Aziridin wurde wenig trans-Aziridin erhalten.

IR (Film): 3330 m (NH), 1610; s, 1510 s, 1220 s.

Beispiel 20

(±)-Dichloro-[1,2-bis(4-chlor-phenyl)-ethylendiamin]-platin(II) ((±) 4-Cl)

Gelbes Pulver, F. ca. 370°C (Zersetzung)

IR-Spektrum in Kbr: 3210 s, 3190 s, 3100 m ($NH_2$), 1600 m, 1560 m, 1495 s, 1415 m, 1090 s, 1015 s, 825 s, 600 s, 520 m, 320 m (Pt-Cl)

$^1H$—NMR: δ = 5,55 (br, 2 H, NH); 6,28 (br, 2 H, NH); 6,72 (d, 2H, CH); 7,34 (d, J = 8,5 Hz, 4 H, meta H); 7,86 (d, 8,5 Hz, 4H; ortho H).

Das Ausgangsamin wird gemäß Methode C erhalten.

Beispiel 21

meso-Dichloro-[1,2-bis(4-chlor-phenyl)ethylendiamin]-platin(II) (meso 4—Cl)

Gelbes Pulver, F. ca. 315°C (Zersetzung)

IR-Spektrum in Kbr: 3240 s, 3180 s, 3100 m, 3020 m ($NH_2$); 1550 m, 1170 s, 1040 s, 812 s, 770 s, 315 m (Pt-Cl)

$^1H$—NMR: δ = 4,65 (br, 2 H, NH); 5,79 (br, 2 H, NH); 6,25 (d, 2H, CH); 7,34 (d, J = 8 Hz, 4 H, meta H);) 7,64, 7,68 (d, J = 8 Hz, 4 H, ortho H).

Das Ausgangsamin wird gemäß Methode B erhalten.

Beispiel 22

(±)-Dichloro-[1,2-bis(3-chlor-phenyl)ethylendiamin])platin(II) ((± 3-Cl)

Gelbes Pulver

IR-Spektrum in KBr: 3260 s, 3200 s ($NH_2$), 1600 m, 1575 s, 1480 m, 1440 m, 790 s, 720 m, 690 s, 440 w, 320 m (Pt-Cl)

$^1$H—NMR: δ = 5,39 (breit, 2 H, NH); 6,14 (breit, 2 H, NH); 6,62 (d, 2H, CH); 7,29—7,88 (m, 8 H, aromat H).

Das Ausgangsamin kann ausgehend vom 3,3'-Dichlorstilben über das entsprechende cis-1,2-Bis-(3-chlor-phenyl)-aziridin und das threo-1-Azido-2-amino-1,2-bis-(3-chlorphenyl)-ethan analog der Vorschrift erhalten werden, die in der DE—OS 34 05 611, Seiten 17—19 angegeben ist.

Beispiel 23

meso-Dichloro-[1,2-bis(3-chlor-phenyl)-ethylendiamin]-platin(II) (meso 3-Cl)

Reaktionszeit 3 Tage

Gelbes Pulver

IR-Spektrum in Kbr: 3240 bs, 3180 2, 3100 m, 3020 m ($NH_2$), 1550 m, 1170 s, 1040 s, 812 s, 770 s, 315 m (Pt-Cl)

$^1$H—NMR: δ = 4,64 (breit, 2 H, NH); 5,91 (breit, 2 H, NH); 6,26 (d, 2H, CH); 7,27—7,78 (m, 8H, aromatische H).

Das Ausgangsamin ist bekannt.

Beispiel 24

meso-Dichloro-[1,2-bis(3-fluor-phenyl)-ethylendiamin]-platin(II) (meso 3—F)

Synthese unter Zusatz von tertiärem Butanol.

Gelbes Pulver

IR-Spektrum in KBr: 3200 s, 3120 s ($NH_2$), 1630 m, 1600 s, 1500 m, 1460 m, 780 s, 700 s, 520 w, 320 m (Pt-Cl)

$^1$H—NMR: δ = 4,73 (breit, 2 H, NH); 5,87 (breit, 2 H, NH); 6,30 (d, 2H, CH); 7,08—7,16 (m, 2H, aromatische H); 7,28—7,39 (m, 4H, aromatische H); 7,58—7,62 (d, 2H, aromatische H).

Das Ausgangsamin wird gemäß Methode B erhalten.

Beispiel 25

meso-Dichlor-[1,2-bis(4-fluor-phenyl)-ethylendiamin]-platin(II) (meso 4—F)

248 mg (1 mmol) meso-1,2-Bis(4-fluor-phenyl)-ethylendiamin werden in 40 ml 50%igem tertiärem Butanol unter Erwärmen gelöst. Zur Lösung tropft man 415 mg $K_2PtCl_4$, gelöst in 10 ml $H_2O$. Bei 50—60°C wird 5 Stunden unter Lichtausschluß gerührt, anschließend das Produkt abgesaugt mit tertiärem Butanol und $H_2O$ gewaschen und getrocknet.

Gelbes Pulver, F. ca 305°C (Zersetzung)

IR-Spektrum in Kbr: 3250 s, 3190 s, 3120 s ($NH_2$), 1610 s, 1560 s, 1515 s, 1230 s, 805 s, 325 m.

$^1$H—NMR: δ =4,53 (br, 2H, NH); 5,69 (br, 2H, NH); 6,20 (d, 2H, CH); 7,10 (t, $J_{HH}$ = 8,8 Hz, $J_{HF}$ = 8,9, 4 H, meta H); 7,64 (q, $J_{HH}$ = 8,8 Hz, $J_{HF}$ = 5,4 Hz, 4 H, ortho H).

Das Ausgangsdiamin ist bekannt.

Beispiele 26—30

Austausch des Anions X durch andere Anionen

Beispiel 26

Diaquo-meso-1,2-bis(2,6-dichlor-4-hydroxyphenyl)ethylendiamin-platin(II)-sulfat

194 mg (0,3 mmol) meso-1,2-bis(2,6-dichlor-4-hydroxyphenyl)-ethylendiamin-dichloro-platin(II)-Komplex werden mit 93 mg (0,3 mmol) Silbersulfat in 60 ml Wasser im Ultraschallbad suspendiert und 3 Tage bei 50°C unter Lichtausschluß gerührt. Das sich gebildete AgCl und nicht umgesetzter meso-1,2-Bis(2,6-dichlor-4-hydroxyphenyl)ethylendiamin-dichloro-platin(II)-Komplex werden über einen Membranfilter abgesaugt. Nach dem Absaugen verbleibt eine wäßrige Lösung, die an der Ölpumpe zur Trockene eingeengt wird. Der Rückstand wird mit absolutem Methanol digeriert, wobei der Komplex in Lösung geht uns so vom nicht umgesetzten $Ag_2SO_4$ abgetrennt werden kann. Die methanolische Lösung wird möglichst weit eingeengt und der Komplex mit Diisopropylether ausgefällt. Der Niederschlag wird abgesaugt, mit viel Diisopropylether gewaschen und über $P_2O_5$ bei Raumtemperatur getrocknet.

Weißes Pulver, in Wasser löslich; Ausbeute: 80 mg (38% der Theorie).

Beispiel 27

Diaquo-meso-1,2-bis(4-fluorphenyhl)ethylendiamin-platin(II)-sulfat

514 mg (1 mmol) des entsprechenden Dichloro-Komplexes werden mit 310 mg (1 mmol) Silbersulfat in 60 ml Wasser im Ultraschallbad suspendiert und 3 Tage bei 50°C unter Lichtausschluß gerührt. Das sich gebildete AgCl und nicht umgesetzter Dichloro-Komplex werden über einen Membranfilter abgesaugt. Es verbleibt eine gelbe Lösung, die an der Ölpumpe zur Trockene eingeengt wird. Der Rückstand wird mit absolutem Methanol digeriert, wobei der Komplex in Lösung geht und so vom nicht-umgesetzten $Ag_2SO_4$ abfiltriert werden kann. Die methanolische Lösung wird eingeengt und der Komplex mit Diisopropylether

ausgefällt. Der Niederschlag wird abgesaugt, mit viel Diisopropylether gewaschen und über $P_2O_5$ bei Raumtemperatur getrocknet.

Weißes Pulver; Ausbeute: 307 mg (52% der Theorie).

Die folgenden Sulfate wurden analog dem oben be schriebenen Diaquo-meso-1,2-bis-(4-Fluor-phenyl)-ethylendiamin-platin(II)-sulfat hergestellt:

Beispiel 28

Diaquo-d,l-1,2-bis(4-fluorphenyl)ethylendiamin-platin(II)-sulfat

Ansatz: 514 mg (1 mmol) des entsprechenden Dichloro-Komplexes, 310 mg (1 mmol ($Ag_2SO_4$

Weißes Pulver; Ausbeute: 160 mg (26% der Theorie).

Beispiel 29

Diaquo-meso-1,2-bis(2-fluor-4-hydroxyphenyl)ethylendiaminplatin(II)-sulfat

Ansatz: 526 mg (1 mmol) des entsprechenden Dichloro-Komplexes, 310 mg (1 mmol) $Ag_2SO_4$

Weißes Pulver;

Ausbeute: 600 mg (98% der Theorie).

Beispiel 30

Diaquo-meso-1,2-bis(2,6-dichlor-4-hydroxyphenyl) ethylendiamin-platin(II)-sulfat

Ansatz: 194 mg (0,3 mmol) des entsprechenden Dichloro-Komplexes 93 mg (0,3 mmol) $Ag_2SO_4$

Weißes Pulver; Ausbeute: 80 mg (38% der Theorie).

Beispiele für pharmazeutische Zubereitungen

Beispiel (Lyophilisat):

In 800 ml Wasser für Injektionszwecke werden unter Rühren 10 g D-Mannit und 3 g Diaquo-meso-[1,2-bis(2,6-dichlor-4-hydroxyphenyl)-ethylendiamin]-Platin(II)-sulfat gelöst und mit Wasser für Injektionszwecke das Volumen auf 1 Liter aufgefüllt.

Diese Lösung wird unter aseptischen Bedingungen über ein Membranfilter von 0,22 µm Porenweite sterilfiltriert und zu 10 ml in 15 ml Injektionsflaschen der hydrolytischen Klasse I abgefüllt. Die Flaschen werden mit einem Gefriertrocknungsstopfen versehen und in einer geeigneten Anlage lyophilisiert. Nach der Trocknung begast man mit sterilem, getrocknetem Stickstoff und verschließt die Flaschen in der Anlage. Die Stopfen werden durch eine Bördelkappe gesichert.

Für die intravenöse Anwendung wird das Lyophilisat in 10 ml physiologischer Kochsalzlösung rekonstituiert. 1 ml Lösung enthält 3 mg Wirkstoff.

Beispiel (Tabletten):

200 g d,l-Dichloro-[1,2-bis-(2,6-dichloro-4-hydroxyphenyl)-ethylendiamin])-Platin(II)(entsprechend Beispiel 20), 500 g Lactose, 360 g mikrokristalline Cellulose, 130 g Maisstärke und 10 g Magnesiumstearat werden durch ein Sieb mit einer Maschenweite von 0,8 mm gegeben und homogenisiert. Diese Masse wird in bekannter Weise zu Tabletten von 120 mg gepreßt. 1 Tablette enthält 20 mg Wirkstoff.

Beispiel (Überzogene Tabletten)

Zur Herstellung von Filmtabletten werden Tabletten entsprechend dem vorangegangenen Beispiel in bekannter Weise mit Hilfe einer Sprüheinrichtung mit einem Magenoder Dünndarm-löslichen Überzug versehen, der aus einem geeigneten polymeren Filmbildner wie zum Beispiel Estern von Acrylaten oder Methacrylaten und geeigneten Hilfsstoffen wie Netzmitteln, Weichmachern, Farbstoffen, Gleitmitteln usw. bestehen kann. Die Tabletten können auch in üblicher Weise zu Dragees verarbeitet werden.

**Patentansprüche**

1. (1,2-Diphenyl-ethylendiamin)-platin(II)-Komplexe der allgemeinen Formel

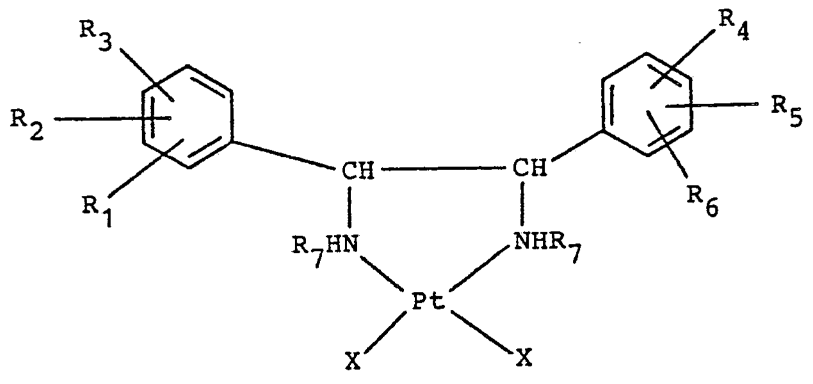

I

worin $R_7$ Wasserstoff oder $C_1$—$C_6$-Alkyl bedeutet und $R_2$ ein Halogenatom ist und die Reste $R_1$, $R_3$, $R_4$, $R_5$ und $R_6$ gleich oder verschieden sind und Wasserstoff, Halogen, Trihalogenmethyl, $C_1$—$C_6$-Alkyl, Hydroxy, $C_1$—$C_6$-Alkoxy oder eine gegebenenfalls durch Halogen oder $C_1$—$C_6$-Alkansulfonyloxy substituierte $C_2$—$C_6$-Alkanoyloxygruppe bedeuten, und X für das Äquivalent eines physiologisch verträglichen Anions steht.

2. Verfahren zur Herstellung von (1,2-Diphenyl-ethylendiamin)-platin(II)-Komplexen der allgemeinen Formel

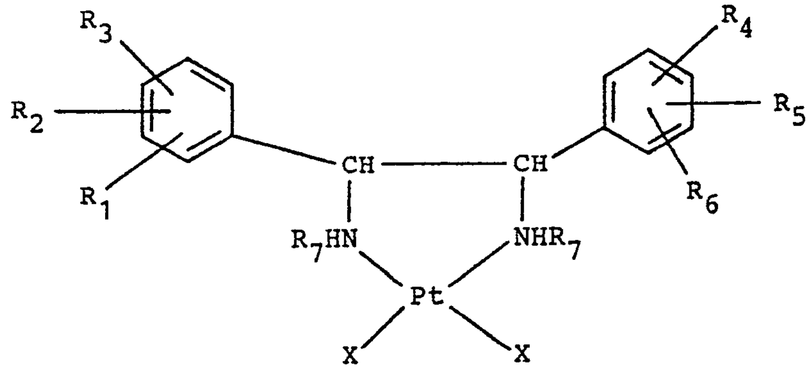

I

worin $R_7$ Wasserstoff oder $C_1$—$C_6$-Alkyl bedeutet und $R_2$ ein Halogenatom ist und die Reste $R_1$, $R_3$, $R_4$, $R_5$ und $R_6$ gleich oder verschieden sind und Wasserstoff, Halogen, Trihalogenmethyl, $C_1$—$C_6$-Alkyl, Hydroxy, $C_1$—$C_6$-Alkoxy oder eine gegebenenfalls durch Halogen oder $C_1$—$C_6$-Alkansulfonyloxy substituierte $C_2$—$C_6$-Alkanoyloxygruppe bedeuten, und X für das Äquivalent eines physiologisch verträglichen Anions steht, dadurch gekennzeichnet, daß man eine Tetrahalogeno-platin(II)-säure, ein Tetrahalogeno-platin(II)-Komplexsalz miot zwei einwertigen oder einem zweiwertigen Kation oder ein Platin(II)-halogenid mit einer Verbindung der Formel

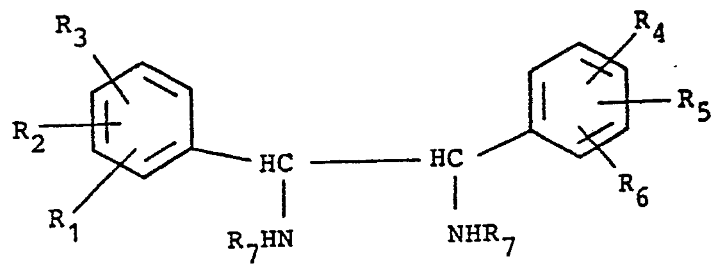

II

oder einem Säureadditionssalz der Verbindung II, wobei die Reste $R_1$, $R_2$, $R_3$, $R_4$, $R_5$, $R_6$ und $R_7$ die angegebenen Bedeutungen haben, umsetzt, gegebenenfalls in vorhandene freie phenolische Hydroxygruppen eine gegebenenfalls durch Haloen oder $C_1$—$C_4$-Alkansulfonyloxy substituierte $C_2$—$C_6$-Alkanoylgruppe einführt, und gegebenenfalls in einer erhaltenen Verbindung der Formel I den Rest X beziehungsweise die Reste X gegen andere physiologisch verträgliche Anionen austauscht.

3. Arzneimittel, enthaltend eine Verbindung der allgemeinen Formel I neben üblichen Träger und/oder Verdünnungs- beziehungsweise Hilfsstoffen.

4. Verfahren zur Herstellung eines Arzneimittels, dadurch gekennzeichnet, daß eine Verbindung der allgemeinen Formel I mit gebräuchlichen pharmazeutischen Trägerstoffen oder Verdünnungsmitteln beziehungsweise sonstigen Hilfsstoffen zu pharmazeutischen Zubereitungen verarbeitet beziehungsweise in eine therapeutisch anwendbare Form gebracht wird.

5. Verwendung von Verbindungen der allgemeinen Formel I zur Herstellung von Arzneimitteln.

**Revendications**

1. Complexes de 1-2-diphényléthylènediamine et de platine(II) de formule générale

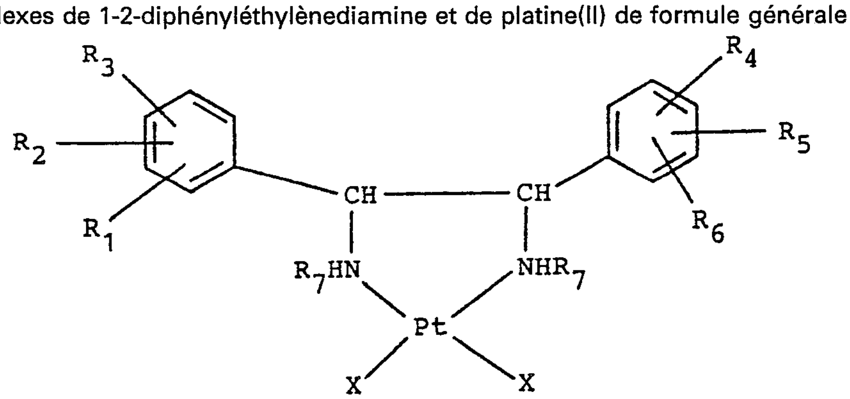

I

dans laquelle $R_7$ représente un atome d'hydrogène ou un groupement alkyle en $C_1$—$C_6$, $R_2$ est un atome d'halogène, les radicaux $R_1$, $R_3$, $R_4$, $R_5$ et $R_6$ sont semblables ou différents et sont chacun un atome d'hydrogène, d'halogène, un groupement trihalogénométhyle, alkyle en $C_1$—$C_6$, hydroxy, alcoxy en $C_1$—$C_6$ ou un groupement alcanoyloxy en $C_2$—$C_6$ éventuellement substitué par un atome d'halogène ou par un groupement $C_1$—$C_4$-alcanesulfonyloxy, et X est mis pour l'équivalent d'un anion acceptable physiologiquement.

2. Procédé de préparation de complexes de 1-2-diphényléthylènediamine et de platine(II) de formule générale

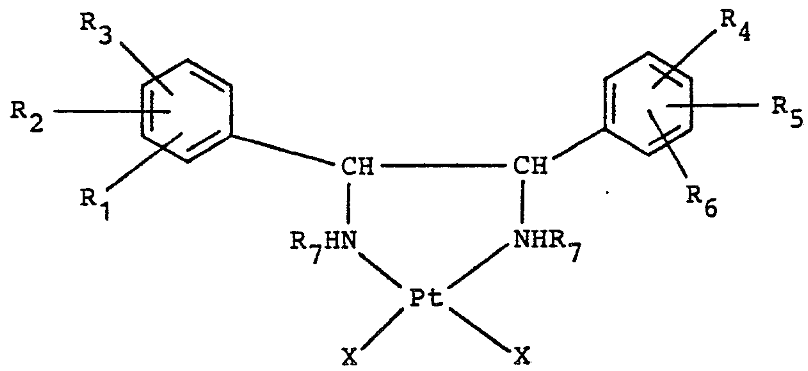

I

dans laquelle $R_7$ représente un atome d'hydrogène ou un groupement alkyle en $C_1$—$C_6$, $R_2$ est un atome d'halogène, les radicaux $R_1$, $R_3$, $R_4$, $R_5$, et $R_6$ sont semblables ou différents et sont chacun un atome d'hydrogène, d'halogène, un groupement trihalogénométhyle, alkyle en $C_1$—$C_6$, hydroxy, alcoxy en $C_1$—$C_6$ ou un groupement alcanoyloxy en $C_2$—$C_6$ éventuellement substitué par un atome d'halogène ou par un groupement en $C_1$—$C_4$-alcanesulfonyloxy, et X est mis pour l'équivalent d'un anion acceptable physiologiquement, caractérisé en ce qu'on fait réagir un acide tétrahalogénoplatineux, un sel de complexe tétrahalogénoplatineux comportant deux cations monovalents ou un cation bivalent ou un halogénure platineux avec un composé de formule

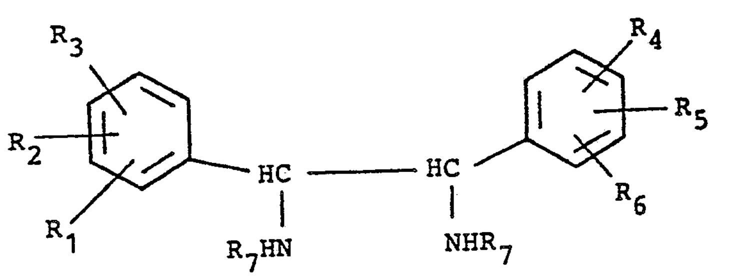

II

ou avec un sel d'acide d'addition du composé II, les radicaux $R_1$, $R_2$, $R_3$, $R_4$, $R_5$ $R_6$ et $R_7$ ayant les significations indiquées, on introduit le cas échéant, dans des groupements hydroxy phénoliques libres présents, un groupement alcanoyle en $C_2$—$C_6$ éventuellement substitué par un atome d'halogène ou un groupement $C_1$—$C_4$-alcanesulfonyloxy et on remplace le cas échéant, dans un composé de formule I obtenu, le radical X ou les radicaux X par d'autres anions acceptables physiologiquement.

3. Médicament contenant un composé de formule générale I, outre des excipients et/ou des diluants ou adjuvants usuels.

4. Procédé de préparation d'un médicament, caractérisé en ce qu'un composé de formule générale I est transforme en préparations pharmaceutiques ou mis sous une forme utilisable en thérapeutique avec des excipients, des diluants ou d'autres adjuvants pharmaceutiques usuels.

5. Utilisation de composés de formule générale I pour la préparation de médicaments.

**Claims**

1. (1,2-Diphenylethylenediamine)-platinum(II) complexes corresponding to the following general formula

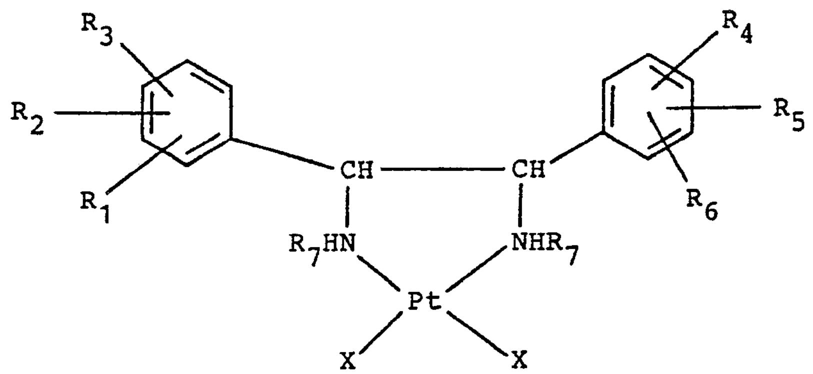

I

in which $R_7$ is hydrogen or $C_1$—$C_6$alkyl and $R_2$ is a halogen atom and $R_1$, $R_2$, $R_3$, $R_4$, $R_5$ and $R_6$ may be the same or different and represent hydrogen, halogen, trihalomethyl, $C_1$—$C_6$alkyl, hydroxy, $C_1$—$C_6$alkoxy or an optionally halogen or $C_1$—$C_4$-alkanesulfonyloxy-substituted $C_2$—$C_6$ alkanoyloxy group and X is the equivalent of a physiologically acceptable anion.

2. A process for the preparation of (1,2-diphenylethylenediamine)-platinum(II) complexes corresponding to the following general formula

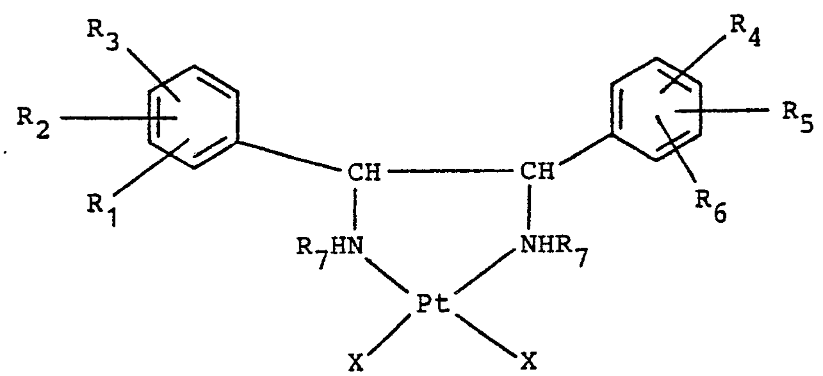

I

in which $R_7$ is hydrogen or $C_1$—$C_6$alkyl and $R_2$ is a halogen atom and $R_1$, $R_2$, $R_3$, $R_4$, $R_5$ and $R_6$ may be the same or different and represent hydrogen, halogen, trihalomethyl, $C_1$—$C_6$alkyl, hydroxy, $C_1$—$C_6$alkoxy or an optionally halogen or $C_1$—$C_4$-alkanesulfonyloxy-substituted $C_2$—$C_6$ alkanoyloxy group and X is the equivalent of a physiologically acceptable anion, characterized in that a tetrahaloplatinum(II) acid, a tetrahaloplatinum(II) complex salt containing two monovalent or one divalent or a platinum(II) halide is reacted with a compound corresponding to the following formula

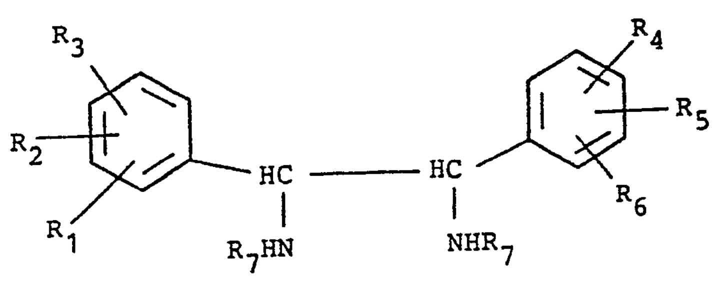

II

or an acid addition salt of compound II, $R_1$, $R_2$, $R_3$, $R_4$, $R_5$, $R_6$ and $R_7$ having the meanings defined above, an optionally halogen- or $C_1$—$C_4$-alkanesulfonyloxy-substituted $C_2$—$C_6$ alkanoyl group is optionally introduced into free phenolic hydroxy groups present and the substituent X or the substituents X in the resulting compound of formula I is/are optionally replaced by other physiologically acceptable anions.

3. A medicament containing a compound corresponding to general formula I in addition to standard excipients and/or diluents or auxiliaries.

4. A process for the production of a medicament, characterized in that a compound corresponding to general formula I is processed to pharmaceutical preparations or brought into a therapeutically usable form with standard pharmaceutical excipients or diluents or other auxiliaries.

5. The use of the compounds corresponding to general formula I for the production of medicaments.